(19) Europäisches Patentamt
European Patent Office
Office européen des brevets

(11) EP 4 023 248 A1

(12) **EUROPEAN PATENT APPLICATION**
published in accordance with Art. 153(4) EPC

(43) Date of publication:
**06.07.2022 Bulletin 2022/27**

(21) Application number: **20857576.1**

(22) Date of filing: **28.08.2020**

(51) International Patent Classification (IPC):
***A61K 48/00*** (2006.01) ***A61P 13/00*** (2006.01)
***A61P 13/02*** (2006.01) ***A61P 13/10*** (2006.01)
***A61P 35/00*** (2006.01) ***A61P 43/00*** (2006.01)
***C12N 5/10*** (2006.01) ***C12N 15/12*** (2006.01)
***C12N 15/85*** (2006.01) ***A61K 35/22*** (2015.01)
***A61K 35/76*** (2015.01)

(52) Cooperative Patent Classification (CPC):
**A61K 35/22; A61K 35/76; A61K 48/00; A61P 13/00; A61P 13/02; A61P 13/10; A61P 35/00; A61P 43/00; C12N 5/10; C12N 15/85**

(86) International application number:
**PCT/JP2020/032644**

(87) International publication number:
**WO 2021/039972 (04.03.2021 Gazette 2021/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.08.2019 JP 2019159070**

(71) Applicant: **Cellaxia Inc.**
**Tokyo 103-0012 (JP)**

(72) Inventors:
- **INOUE, Yuta**
  **Kyoto-shi, Kyoto 602-8566 (JP)**
- **MAZDA, Osam**
  **Kyoto-shi, Kyoto 602-8566 (JP)**
- **KISHIDA, Tsunao**
  **Kyoto-shi, Kyoto 602-8566 (JP)**
- **UKIMURA, Osamu**
  **Kyoto-shi, Kyoto 602-8566 (JP)**
- **SEKI, Makoto**
  **Tokyo 103-0012 (JP)**

(74) Representative: **Heubeck, Christian**
**Weickmann & Weickmann**
**Patent- und Rechtsanwälte PartmbB**
**Postfach 860 820**
**81635 München (DE)**

# (54) UROTHELIAL CELL INDUCTION AGENT AND METHOD FOR INDUCING UROTHELIAL CELLS

(57) The present invention aims to provide a method for preparing urothelial cells that can be applied to the treatments of urologic diseases, particularly, diseases caused by urothelial cell damage, diseases caused by loss of urothelial cells and dysfunction, and the like, urothelial cells prepared by said method, and a medium for inducing (generating) urothelial cells. Urothelial cells prepared by a method for inducing a urothelial cell, including a step of introducing at least one member selected from the group consisting of
FOXA1 (Forkhead box A1) gene or an expression product thereof, TP63 (tumor protein P63) gene or an expression product thereof, MYCL (L-Myc) gene or an expression product thereof, and
KLF4 (Kruppel-like factor 4) gene or an expression product thereof
to a mammalian somatic cell as an exogeneous factor can be applied to the treatment of urologic diseases.

EP 4 023 248 A1

SEQUENCE LISTING

<110> CellAxia Inc.

<120> Inducing agent for urothelial cell and Induction method of urothelial cell

<130> 093046

<150> JP2019-159070
<151> 2019-08-30

<160> 8

<170> PatentIn version 3.5

<210> 1
<211> 41
<212> DNA
<213> Artificial Sequence

<220>
<223> PCR primer specific for FOXA1

<400> 1
cagcagtgtg gtggtacggg atgttaggaa ctgtgaagat g 41

<210> 2
<211> 37
<212> DNA
<213> Artificial Sequence

<220>
<223> PCR primer specific for FOXA1

<400> 2
accggcgctc agctggctag gaagtgttta ggacggg 37

<210> 3
<211> 38
<212> DNA
<213> Artificial Sequence

<220>
<223> PCR primer specific for IRF1

<400> 3
cagtgtggtg gtacgggatg cccatcactc ggatgcgc 38

<210> 4
<211> 32
<212> DNA
<213> Artificial Sequence

<220>
<223> PCR primer specific for IRF1

<400> 4
accggcgctc agctggaccg gcgctcagct gg 32

```
<210>  5
<211>  38
<212>  DNA
<213>  Artificial Sequence

<220>
<223>  PCR primer specific for TP63

<400>  5
cagtgtggtg gtacgggatg aattttgaaa cttcacgg                        38


<210>  6
<211>  37
<212>  DNA
<213>  Artificial Sequence

<220>
<223>  PCR primer specific for TP63

<400>  6
accggcgctc agctggctat cactccccct cctcttt                         37


<210>  7
<211>  38
<212>  DNA
<213>  Artificial Sequence

<220>
<223>  PCR primer specific for Sonic hedgehog

<400>  7
cagtgtggtg gtacgggatg ctgctgctgg cgagatgt                        38


<210>  8
<211>  37
<212>  DNA
<213>  Artificial Sequence

<220>
<223>  PCR primer specific for Sonic hedgehog

<400>  8
accggcgctc agctggctag ctggacttga ccgccat                         37
```

## Description

[Technical Field]

The present invention mainly relates to a urothelial cell induction method. More particularly, it relates to a method for inducing urothelial cells by direct reprogramming, and an agent for converting somatic cells to urothelial cells.

[Background Art]

Urothelial cells form urothelium on the surface of the urinary tract that is composed of renal pelvis, ureter, urinary bladder and proximal urethra. The urothelium contracts or expands depending on the amount of urine stored and provides a strong barrier to urine exudation and bacterial invasion. Both or either of loss of urothelial cells and dysfunction are/is associated with urinary tract diseases such as bladder cancer, overactive bladder, congenital bladder anomalies, bladder injury, interstitial cystitis, neurogenic bladder, and contracted bladder. Damaged urothelium in patients could be treated by transplantation of autologous intestine or colon tissue segments (e.g., surgical method of removing intestinal epithelium and transplanting same as bladder epithelium). However, these gastrointestinal tissues easily resorb urine, causing many problems including cancer formation, metabolic acidosis, infection, stone formation, etc.

As current treatments for Hannah-type interstitial cystitis, which is one type of interstitial cystitis, injection of DMSO and xylocaine into the bladder and forcible expansion of the bladder (bladder expansion) are performed. However, although short-term results are good, long-term results are poor, and there is no fundamental treatment method as the situation stands.

In view of such problems, a lot of efforts have been devoted to search for another source of the cells applicable to transplantation instead of gastrointestinal tissue segments. Autologous transplantation of bladder tissue expanded by culture using a collagen-polyglycolic acid scaffold has been reported. This achieved sufficient results for short-terms after the transplantation, but this method has not been widely used because the autologous bladder tissue may become dysfunctional after long-term follow-up. A method for inducing urothelium from non-urothelial immature stem cells has been reported. A method of co-culturing mesenchymal stem cells derived from human fetal bone marrow with urothelial cells of patient (Non Patent Literature 1) and a method of culturing human adipose-derived stem cells by adding culture supernatant of urothelial cells (Non Patent Literature 2) have been reported. However, these cells were hardly applicable to clinical use, because the procedures required autologous urothelial cells. In addition, methods of generating urothelial cells from human ES cells and induced pluripotent stem (iPS) cells have been reported (Non Patent Literatures 3, 4). However, transplantation of cells derived from human pluripotent stem cells could cause teratoma formation.

On the other hand, the direct conversion (direct reprogramming) technology that induces differentiation of tissue cells from another somatic cell lineage without passing through a pluripotent state is known. The present inventors have so far established and reported a direct reprogramming technique from somatic cell (e.g., fibroblast) into osteoblast (Patent Literature 1, Non Patent Literature 5), into brown adipocyte (Patent Literature 2, Non Patent Literature 6), into Schwann cell (Patent Literature 3, Non Patent Literature 7) and into myoblast (Patent Literature 4, Non Patent Literature 8). However, conversion of somatic cells into urothelial cells has not been reported.

[Citation List]

[Patent Literatures]

[PTL 1]
WO 2015/012377
[PTL 2]
WO 2014/010746
[PTL 3]
WO 2016/039462
[PTL 4]
WO 2018/124292

[Non Patent Literatures]

[NPL 1]
Ning J, et al., (2011) Cytotechnology 63(5):531-539.
[NPL 2]
Shi JG, et al., (2012) Cell Tissue Res 347(3):737-746.
[NPL 3]
Osborn SL, et al., (2014) Stem Cell Transl Med 3(5):610-619.
[NPL 4]
Kang M, et al., (2014) Int J Mol Sci 15(5):7139-7157.
[NPL 5]
Yamamoto K, et al., (2015) Proc Natl Acad Sci USA 112 (19) :6152-6157.
[NPL 6]
Kishida T, et al., (2015) Stem Cell Reports 5(4):569-581.
[NPL 7]
Sowa Y, et al., (2017) Stem Cells Transl Med 6(4):1207-1216.
[NPL 8]
Wakao J, et al., (2017) Biochem Biophys Res Commun 488(2):368-373.

[Summary of Invention]

[Technical Problem]

The present invention aims to provide a method for generating urothelial cells that can be applied to the treatments of urologic diseases, particularly, diseases caused by urothelial cell damage, diseases caused by loss of urothelial cells and dysfunction, and the like, urothelial cells generated by said method, and a medium for inducing urothelial cells that is suitable for the method.

[Solution to Problem]

In view of the above-mentioned problem, the present inventors have conducted intensive studies based on the findings so far. Specifically, they attempted to generate urothelial cells from somatic cells without going through a pluripotent state by direct reprogramming technique. Direct reprogramming can be achieved by transducing genes encoding transcription factors that play an important role in the differentiation stage of target cells and genes that promote reprogramming of somatic cells. The present inventors have found that human fibroblasts can be directly converted into urothelial cells by introducing some genes, established a procedure of the conversion, and analyzed phenotypes and functions of the resultant directly converted urothelial cells (dUC), and completed the present invention.
Accordingly, the present invention provides the following.

[1] A method for introducing a urothelial cell, comprising a step of introducing at least one member selected from the group consisting of

FOXA1 (Forkhead box A1) gene or an expression product thereof, TP63 (tumor protein P63) gene or an expression product thereof, MYCL (L-Myc) gene or an expression product thereof, and
KLF4 (Kruppel-like factor 4) gene or an expression product thereof
to a mammalian somatic cell as an exogeneous factor.

[2] The method of the above-mentioned [1], wherein the exogeneous factor comprises FOXA1 gene or an expression product thereof, and TP63 gene or an expression product thereof.
[3] The method of the above-mentioned [1], wherein the exogeneous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof

(vii) FOXA1 gene or an expression product thereof, TP63 gene or an expression product thereof, MYCL gene or an expression product thereof, and KLF4 gene or an expression product thereof.

[4] The method of any of the above-mentioned [1] to [3], wherein the aforementioned somatic cell is human fibroblast.
[5] A urothelial cell derived from a mammalian somatic cell and having at least one member selected from the group consisting of

FOXA1 gene or an expression product thereof,
TP63 gene or an expression product thereof,
MYCL gene or an expression product thereof, and
KLF4 gene or an expression product thereof, as an exogeneous factor.

[6] The cell of the above-mentioned [5], wherein the exogeneous factor comprises FOXA1 gene or an expression product thereof, and TP63 gene or an expression product thereof.
[7] The cell of the above-mentioned [5], wherein the exogeneous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

[8] The cell of any of the above-mentioned [5] to [7], wherein the aforementioned somatic cell is human fibroblast.
[9] An agent for converting a mammalian somatic cell to a urothelial cell, comprising at least one member selected from the group consisting of

FOXA1 gene or an expression product thereof,
TP63 gene or an expression product thereof,
MYCL gene or an expression product thereof, and
KLF4 gene or an expression product thereof, as an exogeneous factor.

[10] The agent of the above-mentioned [9], wherein the exogeneous factor comprises FOXA1 gene or an expression product thereof, and TP63 gene or an expression product thereof.
[11] The agent of the above-mentioned [9], wherein the exogeneous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

[12] The agent of any of the above-mentioned [9] to [11], wherein the aforementioned somatic cell is human fibroblast.
[13] A vector for converting a mammalian somatic cell to a urothelial cell, comprising at least one member selected from the group consisting of

FOXA1 gene or an expression product thereof,

TP63 gene or an expression product thereof,
MYCL gene or an expression product thereof, and
KLF4 gene or an expression product thereof, as an exogeneous factor.

[14] The vector of the above-mentioned [13], wherein the exogeneous factor comprises FOXA1 gene or an expression product thereof, and TP63 gene or an expression product thereof.
[15] The vector of the above-mentioned [13], wherein the exogeneous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

[16] The vector of any of the above-mentioned [13] to [15], wherein the aforementioned somatic cell is human fibroblast.
[17] A therapeutic agent for a urologic disease, comprising a cell obtained by the method of any of the above-mentioned [1] to [4], a urothelial cell of any of the above-mentioned [5] to [8], an agent of any of the above-mentioned [9] to [12], or a vector of any of the above-mentioned [13] to [16].
[18] The therapeutic agent of the above-mentioned [17], wherein the urologic disease is a urinary disease.
[19] The therapeutic agent of the above-mentioned [18], wherein the urinary disease is at least one member selected from the group consisting of bladder cancer, overactive bladder, congenital bladder anomalies, bladder injury, interstitial cystitis, neurogenic bladder and contracted bladder.
[20] A medium for urothelial cell induction, comprising 3-isobutyl 1-methylxanthine (IBMX) and epidermal growth factor (EGF) .

[Advantageous Effects of Invention]

According to the present invention, urothelial cells can be generating from somatic cells in a short period of time by direct reprogramming. Since the urothelial cells can be easily derived from the somatic cells of the person who receives transplantation, problems such as immunological rejection and the like do not occur when the obtained urothelial cells are transplanted. In addition, since urothelial cells can be directly induced from somatic cells without going through pluripotent states such as iPS cells and ES cells, problems caused by pluripotent stem cells such as canceration and the like can be avoided.

[Brief Description of Drawings]

[Fig. 1]
Fig. 1 is a diagram showing a cell conversion method. aHDFs were seeded on a 12-well plate at a concentration of $3\times10^4$ cells/well and cultured in a standard medium. The next day (day 0), the gene of interest was introduced with a retroviral vector. Unless otherwise specified, the cells were cultured in the standard medium until day 4 from introduction, then the medium was replaced with CnT-Prime medium, and the cells were cultured while changing the medium once every 3-4 days. Real-time RT-PCR, phase contrast microscope observation, and immunocyte chemical analysis were performed on the 21st day (day 21).
[Fig. 2]
Fig. 2 exhibits diagrams showing the investigation results of whether fibroblasts are converted to urothelial cells by gene introduction using a transcription factor associated with urothelial cells. aHDFs were seeded on an uncoated 12-well plate, and FOXA1 (F), IRF1(I), TP63 (T) and/or sonic hedgehog (SHH) (H) genes were introduced (in lower part of A, the black parts indicate infection with each retroviral vector). The cells were cultured in the standard medium for the period of days 1-3 and CnT-Prime medium for the period of days 4-21. (A) RNA was extracted from the cells, and the mRNA expression level of the UPK1b gene was measured by real-time RT-PCR. The values shown by mean±SD were normalized by β-actin mRNA expression and shown relative to the value of the control without gene introduction as 1.0 (N=3). (B) Representative phase-contrast microscopic images of cells, imaging magnification: x10. FITH (FOXA1, IRF1, TP63, SHH), FIT (FOXA1, IRF1, TP63), FIH (FOXA1, IRF1, SHH), FTH (FOXA1, TP63, SHH) and ITH (IRF1, TP63, SHH).

[Fig. 3]

Fig. 3 is a graph showing the investigation results of whether fibroblasts are converted to urothelial cells by gene introduction using a transcription factor associated with urothelial cells and a reprogramming gene in combination. aHDFs were seeded on a laminin-coated 12-well plate, and F, I, T, H, POU5F1(P), KLF4(K) and/or MYCL(L) genes were introduced (in lower parts of the Figure, the black parts and gray parts indicate infection with each corresponding retroviral vector). The cells were cultured in the standard medium for the period of days 1-3 and CnT-Prime medium for the period of days 4-21. On day 21, total RNA was extracted from each well, and the UPK1b mRNA level was measured by real-time RT-PCR. The values were normalized by β-actin mRNA expression and shown relative to the value of the control without gene introduction as 1.0 (N=1). Some Combinations of the transcription factors promoted UPK1b expression in aHDFs.

[Fig. 4]

Fig. 4 is a diagram showing the investigation results of whether fibroblasts are converted to urothelial cells by gene introduction using a transcription factor associated with urothelial cells and a reprogramming gene in combination. Gene introduction of F, T, P, K, L caused formation of urothelial cell colony expressing UPK1b. aHDFs were seeded on a laminin-coated 12-well plate, and the gene group shown in lower parts of (A), the black parts and gray parts, were introduced. The cells were cultured in the standard medium for the period of days 1-3 and CnT-Prime medium for the period of days 4-21. (A) RNA was extracted from the cells, and the expression level of the UPK1b gene was measured by real-time RT-PCR. mRNA extracted from HUCs was also measured in the same manner. The values shown by mean±SD were normalized by β-actin mRNA expression and shown relative to the value of the control without gene introduction as 1.0 (N=3). (B) Representative phase-contrast microscopic images of cells, imaging magnification: x20. FIPKL (FOXA1, IRF1, POU5F1, KLF4, MYCL), FTPKL (FOXA1, TP63, POU5F1, KLF4, MYCL), FHPKL (FOXA1, SHH, POU5F1, KLF4, MYCL) and FPKL (FOXA1, POU5F1, KLF4, MYCL).

[Fig. 5]

Fig. 5 is a diagram showing the investigation results of whether fibroblasts are converted to urothelial cells by gene introduction using a transcription factor associated with urothelial cells and a reprogramming gene in combination. aHDFs were seeded on a laminin-coated 12-well plate, and the gene group shown in lower parts of (A), the black parts and gray parts, were introduced. The cells were cultured in the standard medium for the period of days 1-3 and CnT-Prime medium for the period of days 4-21. (A) RNA was extracted from the cells, and the mRNA expression levels of the UPK1b gene and UPK2 gene were measured by real-time RT-PCR. mRNA extracted from HUCs was also measured in the same manner. The values shown by mean±SD were normalized by β-actin mRNA expression and shown relative to the value of the control without gene introduction as 1.0 (N=3). Significant difference between groups was found at $^{*}P<0.05$, $^{***}P<0.001$. (B) Representative phase-contrast microscopic images of cells, imaging magnification: x10. Gene introduction of F, T, K, L was sufficient for conversion to urothelial cells expressing UPK1b, UPK2. FTPKL (FOXA1, TP63, POU5F1, KLF4, MYCL), FTPK (FOXA1, TP63, POU5F1, KLF4), FTLK (FOXA1, TP63, MYCL, KLF4) and FTPL (FOXA1, TP63, POU5F1, MYCL).

[Fig. 6]

Fig. 6 is a diagram showing the investigation results of whether fibroblasts are converted to urothelial cells by gene introduction using a transcription factor associated with urothelial cells and a reprogramming gene in combination. (A) aHDFs were seeded on a laminin-coated 12-well plate. The next day (day 0), a retrovirus vector containing any of FOXA1(F), TP63 (T), MYCL (L), KLF4 (K) genes was introduced into the cells. The introduced genes are indicated with "+". The cells were cultured in the standard medium for the period of days 1-3 and CnT-Prime medium for the period of days 4-21. RNA was extracted from the cells, and the mRNA levels of UPK1b and UPK2 were measured by real-time RT-PCR. $^{*}P<0.05$, $^{***}P<0.001$ (to aHDFs without gene introduction). $^{\#}P<0.05$, RNA $^{\#\#\#}P<0.001$ (to HUCs). As a control, RNA extracted from HUCs was also analyzed. (B, C) aHDFs were cultured on plates without coating (N), or coated with collagen (C), poly-L-lysine (P), laminin (L). After introducing the F, T, L, and K genes with a retrovirus (day 0), the cells were cultured in the same manner as in (A). 21 days after culturing, confocal microscopic image (B) was obtained and real-time RT-PCR analysis (C) was performed. $^{*}P<0.05$, $^{***}P<0.001$ (to N). $^{\#\#}P<0.01$, $^{\#\#\#}P<0.001$ (to HUCs). (D, E) aHDFs were seeded on a plate coated with laminin, the F, T, L, and K genes were introduced with a retroviral vector, and the cells were cultured in standard medium for 1-3 days. Then, the cells were cultured in standard medium (S) or CnT-Prime (C) until day 21, and confocal microscopy (D) and real-time RT-PCR analysis (E) were performed. $^{***}P<0.001$ (to aHDFs without gene introduction and cultured in standard medium). $^{\#\#}P<0.01$, $^{\#\#\#}P<0.001$ (to HUCs). The values indicate mean±SD (n=3).

[Fig. 7] Fig. 7 is a diagram showing how passage of dUCs is influenced by difference in the medium. aHDFs into which F, T, L, and K genes had been introduced were cultured in standard medium for the period of days 1-3, and in each medium shown in the Figure for the period of days 4-21. The images taken by a phase contrast microscope are shown. The bar is 100 μm. Passage of dUCs was successful by the difference in the medium.

[Fig. 8]

Fig. 8 is a diagram showing that cells into which F, T, L, and K were introduced have urothelial cell-like forms. aHDFs were seeded on a laminin-coated 12-well plate, FTLK were introduced thereinto, and the cells were cultured in the standard medium for the period of days 1-3 and CnT-Prime medium for the period of days 4-21. (a) RNA was extracted on the day indicated in the Figure, and mRNAs of UPK1b and UPK2 were measured by real-time RT-PCR. mRNA of HUCs was also measured. $^{**}$P<0.01, $^{***}$P<0.001 (to day 0). $^{###}$P<0.001 (to HUCs). (b) Confocal microscopic images. (c, d) On day 0 and day 21, the cells were stained with the antibodies shown in the Figure and Hoechst 33342. The fluorescence microscopic images (c) and the ratio of positive cells (d) are shown. $^{*}$P<0.05, $^{**}$P<0.01 (to day 0). Each data is shown as mean±SD (N=3). In Fig. d, each column shows the results of UPK1b, UPK2, E-cadherin (CDH1), and keratin 8/18 (KRT8/18) from the left side.

[Fig. 9]

Fig. 9 is a diagram showing the investigation results of the characteristics of dUCs. iPSCs were differentiated into endoderm cells, and then differentiated into iUCs. The dUCs in the Figure are at 21 days after gene introduction. (A) Representative phase-contrast microscopic images of cells shown in the Figure. The bar is 100 μm. (B) Total RNA was extracted from each cell, and measured by real-time RT-PCR. Total mRNA extracted from HUCs was also measured. $^{##}$P<0.01 (to iUCs). The values shown by mean±SD were normalized by β-actin mRNA expression and shown relative to the value of HUCs as 1.0 (N=3). dUCs were more similar to immature urothelial cells than iUCs. In Fig. B, each column shows the results of UPK1a, UPK1b, UPK2, UPK3a and UPK3b from the left side.

[Fig. 10]

Fig. 10 is a diagram showing the investigation results of the expression of pluripotency markers in dUCs. aHDFs were seeded on a laminin-coated plate, F, T, L, and K genes were introduced thereinto, and the cells were cultured in the standard medium for the period of days 1-3 and CnT-Prime medium for the period of days 4-21. (A) RNA was extracted from aHDFs and iPS cells on the day indicated in the Figure. The expression of the genes indicated in the Figure was measured by real-time RT-PCR. The expression of the genes of HUCs was also measured. The values shown by mean±SD were normalized byβ-actin mRNA expression and shown relative to the value of iPS cells as 1.0 (N=3). N.S.: No statistically significant difference as compared with day 0. $^{***}$P<0.001 (to iPS cells). (B) The cells were stained with anti-NANOG antibody and the nuclei were stained with Hoechst 33342. Human iPS cells were also stained as a control (leftmost column). The bar is 100 μm. Expression of pluripotency markers was not detected during the conversion process from aHDFs to dUCs. In Fig. A, each column shows the results of LIN28, POU5F1, SOX2 and NANOG from the left side.

[Fig. 11]

Fig. 11 is a diagram showing the investigation results of the expression of CDX2 and albumin (ALB), AQP5 and CD31 in dUCs. aHDFs were seeded on a laminin-coated plate, F, T, L, and K genes were introduced thereinto, and the cells were cultured in the standard medium for the period of days 1-3 and CnT-Prime medium for the period of days 4-21. RNA was extracted on the day indicated in the Figure, and the expression level of the genes was measured by real-time RT-PCR. mRNAs of human small intestine, liver, salivary gland, human umbilical vein endothelial cell (HUVEC), HUCs were also measured as positive control. The values shown by mean±SD were normalized byβ-actin mRNA expression and shown relative to the value of small intestine (CDX2), liver (albumin; ALB), salivary gland (AQP5) or human umbilical vein endothelial cell (HUVEC)(CD31) as 1.0 (N=3). N.S.: No statistically significant difference as compared with day 0. $^{***}$P<0.001 (to positive control).

[Fig. 12]

Fig. 12 is a diagram showing the investigation results of the influence of medium composition on the passage of dUCs. aHDFs into which F, T, L, and K had been introduced were cultured in CnT-Prime medium, "Uromedium" medium, or "UCM" medium until day 4-21 to prepare dUCs of P0. The cells were detached from the plate, seeded on a new plate and cultured in the medium shown in the Figure for 14 days (D14) or 21 days (D21). The phase contrast microscopic image of each cell is shown. The bar is 100 μm. E, presence or absence of epithelial cell colony formation; N, presence or absence of proliferation of non-epithelial cells. dUCs were successfully passaged with UCM medium.

[Fig. 13]

Fig. 13 is a diagram showing the results of examining "UCM" medium components suitable for the selective proliferation of dUCs. (A) aHDFs into which F, T, L, and K had been introduced were cultured in "Uromedium" medium with modified concentrations of insulin, hydrocortisone, EGF, and IBMX. On Day 21, total RNA was extracted from aHDFs and control aHDFs free of gene introduction, and the gene expression level of UPK2 was measured by real-time RT-PCR. The values shown by mean±SD were normalized byβ-actin mRNA expression and shown relative to the value of aHDFs free of gene introduction as 1.0 (N=3). (B) Phase-contrast microscopic images of aHDFs into which F, T, L, and K were introduced, and cultured for 21 days in "Uromedium" medium in which the concentration of hydrocortisone was modified to 0-fold and the concentration of IBMX was modified to 10-fold. Imaging magnification: x10 (C) Phase-contrast microscopic images of aHDFs into which F, T, L, and K were introduced, and cultured for 21 days in "Uromedium" medium with or without addition of 1 μM tranylcypromine. The bar is 100 μm. (D) Phase-contrast microscopic images of aHDFs into which F, T, L, and K were introduced or not introduced, and cultured for 21 days in "Uromedium" medium or "UCM" medium. The bar is 100 μm.

[Fig. 14]

Fig. 14 is a diagram showing the investigation results of the expression of UPK1b and UPK2 in passaged dUCs. aHDFs into which F, T, L, and K had been introduced were cultured in a selection medium (P0) and then passaged (P1 - P4). (a) Concept diagrams of cell conversion and passage in the experiments performed in Figs. 14-17. (b, c) Immunochemical staining images of dUCs (P0) and dUCs (P4) stained with UPK1b, UPK2, E-cadherin (CDH1) and keratin 8/18 (KRT8/18) (b), and the ratio of UPK1b, UPK2, E-cadherin (CDH1), and keratin 8/18(KRT8/18) positive cells in HDFs, dUCs (P0) and dUCs (P4) (c). The bar is 100 μm. $^{**}$P<0.01, $^{***}$P<0.001 (to aHDFs). †P<0.05, †††P<0.001 (to dUCs (P0)). Each data is shown as mean±SD (N=3). It was found that the dUCs (P0 and P4) express UPK1b and UPK2.

[Fig. 15]

Fig. 15 is a diagram showing the investigation results of the morphology, gene expression and function of passaged dUCs. aHDFs into which F, T, L, and K had been introduced were cultured in standard medium for days 1-3 and UCM medium for days 4-21. The obtained P0 dUCs were detached, resuspended in UCM medium, and seeded on a new plate coated with laminin to obtain P1 dUCs. The passage was repeated to obtain dUCs from P2 to P4. (a) Confocal microscopic images of dUCs from P1 to P4. The bar is 100 μm. (b, c) RNA was extracted and mRNA of the indicated gene was measured by real-time RT-PCR. $^{*}$P<0.05, $^{**}$P<0.01, $^{***}$P<0.001 (compared with aHDFs). $^{\#\#}$P<0.01, $^{\#\#\#}$P<0.001 (to HUCs). †P<0.05, †††P<0.001 (to dUCs (P0)). (d, e) P4 dUCs and aHDFs were cultured in the inner chamber of the transwell, and a permeability assay was performed using FITC-labeled dextran. Confocal microscopic images inside the inner chamber are shown (d). In addition, the ratio of FITC-labeled dextran that leaked into the outer chamber is shown as a relative value % (e) calculated with the control without seeding of cells as 100%. Values are mean±SD (N=3). $^{*}$P<0.001, comparison between groups. dUCs proliferated and increased in number with each passage in UCM medium. In Fig. b, each column shows the results of UPK1b and UPK2 from the left side, and in Fig. c, each column shows the results of UPK1a, UPK3a, UPK3b and E-cadherin (CDH1) from the left side.

[Fig. 16]

Fig. 16 is a diagram showing transcriptome analysis of P2 dUCs. Total RNA was extracted from P2 dUCs (n=3), aHDFs (N=3) and HUCs (N=1), and RNA sequence analysis was performed using Illumina Novaseq 6000 (Illumina). (A) Genes with the highest expression levels of 75% by hierarchical clustering analysis. (B) Hierarchical clustering heat map diagram of the top 50 genes with high expression variation. (C) Volcano Plot gene expression variation analysis comparing dUCs and aHDFs. Several ureteral epithelial cells - and fibroblast-specific genes have been respectively marked.

[Fig. 17A]

Fig. 17A is a diagram showing the results of RNA sequence analysis in P2 dUCs, aHDFs and HUCs. Transcriptome analysis showed high similarity between P2 dUCs and HUCs. Total RNAs of P2 dUCs (N=3), aHDFs (N=3), HUCs (N=1) were sequenced with Illumina Novaseq 6000 as in Fig. 16. Pathway analysis by Gene Set Enrichment Analysis (GESA) was performed using iDEP8.1. GO genesets heatmap comparing P2 dUCs and aHDFs.

[Fig. 17B]

Fig. 17B is a KEGG pathway analysis diagram comparing dUCs and aHDFs. Genes that are highly expressed in dUCs are shown.

[Fig. 18A]

Fig. 18A is a diagram showing the investigation results of the distribution in the bladder tissue of dUCs transplanted into the bladder. (a, b) F, T, L, K, and GFP were introduced into aHDFs with a retroviral vector and cultured in CnT-Prime medium for 21 days. The mRNA expression levels of the illustrated genes were quantified by real-time RT-PCR (a), and representative phase-contrast and fluorescence microscopic images were shown (b). $^{***}$P<0.001 (to aHDFs without gene introduction). $^{\#\#\#}$P<0.001 (to HUCs). N.S.: No statistically significant difference between groups. The bar is 100 μm.

[Fig. 18B]

Fig. 18B is a diagram showing the investigation results of the distribution in the bladder tissue of dUCs transplanted into the bladder. (c, d) aHDFs into which F, T, L, K, and G had been introduced were cultured in standard medium for 4 days and transurethrally transplanted by catheter into the bladder cavity of NOG/SCID mice with interstitial cystitis (c). One week after transplantation, the mice were euthanized. The epithelial tissue of the bladder was stained with the antibody shown in the Figure (1st row, UPK1b; 2nd row, UPK2; 3rd row, KRT8/18; 4th row, CDH1;

5th tow, CDH1; 6th row, CDH1). Nuclei were stained with DAPI. In addition, HE staining of tissue serial sections was performed (d) (N=4 mice/each group). M and L indicate the muscle and the lumen of bladder, respectively. Arrowheads indicate GFP-labeled dUCs. The bar is 100 μm. F, T, L, K, G-introduced GFP-labeled dUCs transplanted into the urothelial mucosa were distributed within the mucosal epithelium of the injured bladder tissue of mice.

[Fig. 19]

Fig. 19 is a diagram showing the investigation results of the tumorigenicity of dUCs. aHDFs into which F, T, L, and K were introduced (4 days after introduction) (+FTLK group), were not introduced (-FTLK group), and T24 cells mixed with Matrigel (Corning) were subcutaneously administered to NOG/SCID mice at a dose of $1\times10^7$ cells/100 μL/mouse (each group N=4). (A) Overall image of mouse. Red triangle indicates a subcutaneous swelling or tumor at the site of administration. (B) The volume of the swollen subcutaneous tissue or tumor was measured every other week. Volumes indicated by value±SD were normalized with data at week 0 of administration (N=4). $^{**}$P<0.01 (to -FTLK group); $^{\#}$P<0.001 (significant difference compared to both -FTLK group and +FTLK group). dUCs did not form tumors in vivo. On the other hand, the bladder cancer cell line T24 formed a tumor.

[Fig. 20]

Fig. 20 is a diagram showing the investigation results of whether fibroblasts are converted to urothelial cells by gene introduction using a transcription factor associated with urothelial cells and a reprogramming gene in combination. aHDFs were seeded on a laminin-coated 12-well plate. The next day (day 0), a retrovirus vector containing any or a combination of FOXA1(F), TP63 (T), MYCL (L), KLF4 (K) genes was introduced into the cells. The cells were cultured in the standard medium for the period of days 1-3 and CnT-Prime medium for the period of days 4-14. RNA was extracted from the cells, and the mRNA levels of UPK1b and UPK2 were measured by real-time RT-PCR. As a control, RNA extracted from HUCs was also analyzed. The values indicate mean (n=3).

[Fig. 21]

Fig. 21 is a diagram showing the barrier function of HUCs in in vitro permeability assay. (A) Confocal microscopic images inside the inner chamber. (B) The ratio of FITC-labeled dextran that leaked into the outer chamber is shown as a relative value % calculated with the control without seeding of cells as 100%. Values are mean±SD (N=3). $^{*}$P<0.01, $^{***}$P<0.001 (to aHDFs). $^{\#}$P<0.05 shows statistically significant difference between groups.

[Description of Embodiments]

The present invention relates to a method for inducing urothelial cells by converting differentiated somatic cells of a mammal into urothelial cells. "Converting" means converting somatic cells into urothelial cells of interest. One of the preferred embodiments of the method of the present invention is a method for converting somatic cells into urothelial cells without going through a step of reprogramming cells initialization represented by the production of iPS cells, which is also called "direct reprogramming" or "direct conversion".

Induction to urothelial cells can be performed either in vitro or in vivo.

Urothelial cell

Urothelial cells (also referred to as transitional epithelial cells) are cells that cover the inside of the urethra from the calyx, renal pelvis, ureter, and bladder to the urethra. In one embodiment, the urothelial cell may be an epithelial cell derived from the bladder.

Somatic cell

In the present invention, the somatic cell to be the target of direct reprogramming is not particularly limited, and may be any as long as it is derived from a mammal. When transplanting urothelial cells into a living body, it is preferable to use somatic cells (autologous cells) derived from the subject who receives transplantation in order to reduce the risk of infection, rejection response, and the like. However, depending on the purpose, urothelial cells prepared in advance from somatic cells of another person or another animal can be used for transplantation instead of autologous cells. Alternatively, urothelial cells may be produced from somatic cells of another person or another animal prepared in advance, and used for transplantation. That is, a bank of urothelial cells may be created and used for transplantation purposes. In such cases, MHC can be typed in advance to reduce the risk of rejection response, and the like. In addition, the cell characteristics, tumorigenicity, and the like of urothelial cells can be confirmed in advance.

In the present specification, examples of the mammal include mouse, rat, hamster, human, dog, cat, monkey, rabbit, bovine, horse, swine and the like, particularly human.

As the somatic cells, somatic cells that can be easily collected from a living body can be used. For example, fibroblast, keratinocyte, mouth cavity mucosa epithelial cell, nasal cavity mucosa epithelial cell, airway mucosa epithelial cell, gastric mucosa epithelial cell, intestinal mucosa epithelial cells, vascular endothelial cell, smooth muscle cell (bladder smooth muscle cell, vascular smooth muscle cell, etc.), adipocyte, gingiva cell (gingiva fibroblast, gingiva epithelial cell), pulp cell, periodontal ligament cell, bone marrow cell, bone marrow-derived stromal cell, leukocyte, lymphocyte, conjunctiva epithelial cell, osteoclast and the like, with preference given to fibroblast, keratinocyte, mouth cavity mucosa epithelial cell, gingiva cell, leukocyte, lymphocyte and the like. More preferably, it is a fibroblast. In the present invention, it is preferable to use the above-mentioned cells collected from a living body.

When induction of somatic cells into urothelial cells is performed in vivo, the target somatic cell is preferably a cell present at a site where supply of urothelial cells is desired. For example, when the target disease is interstitial cystitis, the site where the supply of urothelial cells is desired is the inner surface of the bladder, and therefore, the somatic cell to be the starting material is preferably a cell present on the inner surface of the bladder. Examples of the somatic cell to be the starting material include fibroblast, bladder smooth muscle cell, vascular endothelial cell, vascular smooth muscle cell, lymphocyte present in the bladder, and the like.

Gene or expression product thereof

In the method of the present invention, at least one, preferably two, more preferably three, and even more preferably all four members of FOXA1 (Forkhead box A1) gene or an expression product thereof, TP63 (tumor protein P63) gene or an expression product thereof, MYCL (L-Myc) gene or an expression product thereof, and KLF4 (Kruppel-like factor 4) gene or an expression product thereof is/are introduced as an exogenous factor into somatic cells. As used herein, examples of the "expression product" include mRNAs and proteins of the FOXA1 gene, TP63 gene, MYCL gene and KLF4 gene.

In the method of the present invention, one embodiment of preferable combination of exogeneous factor to be introduced into somatic cells includes a combination of FOXA1 gene or an expression product thereof, and TP63 gene or an expression product thereof.

The method of the present invention wherein the exogeneous factor is selected from the following is also one preferable embodiment.

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

All of these can enhance the expression of UPK2 mRNA.

In the method of the present invention, micro RNA, siRNA, shRNA, and DNA expressing them can also be used in addition to the FOXA1 gene or an expression product thereof, TP63 gene or an expression product thereof, MYCL gene or an expression product thereof, and KLF4 gene or an expression product thereof. In addition, various proteins can also be used concurrently.

All of the above-mentioned genes are highly conserved genes in vertebrates. In the present specification, unless the animal name is specifically indicated, the genes including homologs are referred to. Even if the gene has a mutation, including polymorphism, a gene having a function equivalent to that of a wild-type gene product is also included.

For example, the nucleotide sequences of the FOXA1 gene, TP63 gene, MYCL gene and KLF4 gene of humans (Homo sapiens) and the amino acid sequences of the proteins encoded by the nucleotide sequences are registered with the following accession numbers in the GenBank provided by the National Center for Biotechnology Information (NCBI) (when multiple revisions are registered, it is understood to indicate the latest revision):

human FOXA1 gene cDNA sequence: BC_033890, NM_004496 (e.g., BC_033890.1, NM_004496.5),
human FOXA1 protein amino acid sequence: NP_004487 (e.g., NP_004487.2),
human TP63 gene cDNA sequence: BC_039815, NM_003722 (e.g., BC_039815.1, NM003722.5),
human TP63 protein amino acid sequence: NP_003713 (e.g., NP_003713.3),
human MYCL gene cDNA sequence: NM_001033081, NM_001033082, NM_005376 (e.g., NM_001033081.2, NM_001033082.2, NM_005376.4),
human MYCL protein amino acid sequence: NP_001028253.1, NP_001028254.2, NP_005367.2 (e.g.,

NP_001028253, NP_001028254, NP_0053 67),
human KLF4 gene cDNA sequence: BC_029923, NM_001314052 (e.g., BC_029923.1, NM_001314052.2),
human KLF4 protein amino acid sequence: NP_001300981 (e.g., NP_001300981.1).

Introduction

The method of the present invention can be performed according to a known direct reprogramming method except selection of a specific gene, and can be performed, for example, according to the method of any of the following documents:

1: Direct Reprogramming of Fibroblasts into Functional Cardiomyocytes by Defined Factors; Masaki Ieda, Ji-Dong Fu, Paul Delgado-Olguin, Vasanth Vedantham, Yohei Hayashi, Benoit G. Bruneau, and Deepak Srivastava Cell 142: 375-386, 2010.
2: Direct conversion of fibroblasts to functional neurons by defined factors. Thomas Vierbuchen, Austin Ostermeier, Zhiping P. Pang, Yuko Kokubu, Thomas C. Sudhof & Marius Wernig. Nature 46.
3: 1035-1041, 20103: Induction of human neuronal cells by defined transcription factors. Pang ZP, Yang N, Vierbuchen T, Ostermeier A, Fuentes DR, Yang TQ, Citri A, Sebastiano V, Marro S, Sudhof TC, Wernig M. Nature 476: 220-223, 2011.
4: Generation of hyaline cartilaginous tissue from mouse adult dermal fibroblast culture by defined factors Kunihiko Hiramatsu, Satoru Sasagawa, Hidetatsu Outani, Kanako Nakagawa, Hideki Yoshikawa, and Noriyuki Tsumaki, Journal of Clinical Investigation, 121: 640-657, 2011.
5: Induction of functional hepatocyte-like cells from mouse fibroblasts by defined factors. Pengyu Huang, Zhiying He, Shuyi Ji, Huawang Sun, Dao Xiang, Changcheng Liu, Yiping Hu, XinWang & Lijian Hui,. Nature 475:386-389, 2011.
6: Direct conversion of mouse fibroblasts to hepatocyte-like cells by defined factors. Sayaka Sekiya & Atsushi Suzuki. Nature 475:390-393, 2011.
7: Direct conversion of human fibroblasts into functional osteoblasts by defined factors. Yamamoto K, Kishida T, Sato Y, Nishioka K, Ejima A, Fujiwara H, Kubo T, Yamamoto T, Kanamura N & Mazda O. Proc Natl Acad Sci USA. 112:6152-6157, 2015.
8: Reprogrammed Functional Brown Adipocytes Ameliorate Insulin Resistance and Dyslipidemia in Diet-Induced Obesity and Type 2 Diabetes. Kishida T, Ejima A, Yamamoto K, Tanaka S, Yamamoto T, Mazda O. Stem Cell Reports. 5: 569-581, 2015.
9: Generation of directly converted human osteoblasts that are free of exogenous gene and xenogenic protein. Yamamoto K., Sato Y., Honjo K., Ichioka H., Oseko F., Sowa Y., Yamamoto T., Kanamura N., Kishida T., Mazda O. J Cell Biochem 117:2538-2545, 2016.
10: WO 2014/010746

The contents of the above-mentioned documents 1 - 10 are incorporated herein by reference.
Specifically, it is preferable to incorporate the gene of interest to be an exogenous factor into one or more expression vectors, introduce the expression vector into the target somatic cell, and allow intracellular expression thereof.
A method for introducing the gene includes infecting viral vectors such as retroviral vector, adenoviral vector, lentiviral vector, adeno-associated viral vector, herpes viral vector, Sendai viral vector and the like. In the case of introduction of gene and an expression product thereof, a method for transfecting a plasmid vector, an episomal vector, or a gene expression product (mRNA, protein) with a non-viral vector such as a cationic liposome, a cationic polymer, an electric perforation method, and the like can also be used. In addition, mRNA can also be introduced. All these means used for gene transfer are collectively referred to as a vector in the present specification.
A viral vector is preferable from the aspects of transfer efficiency and stable retention of the transgene, and a plasmid is preferable when the risk of canceration is to be reduced.
In addition, by introducing a gene that serves as a drug selection marker (puromycin resistance, blastsaidin S resistance, neomycin resistance, hygromycin resistance, and the like) together with the gene of interest, and then performing drug selection, the cells expressing the target gene can be selected and then used.
The gene of the present invention may be introduced using a plasmid or a viral vector, for example, a retroviral vector. A viral vector is preferable from the aspects of transfer efficiency and stable retention of the transgene, and a plasmid is preferable when the risk of canceration is to be reduced.
The gene to be introduced into somatic cells can be transcribed by the LTR promoter or may be expressed from another promoter inside the vector. For example, constitutive expression promoters such as CMV promoter, EF-1$\alpha$ promoter, CAG promoter, or the like, or a desired inducible promoter can be utilized. In addition, a chimeric promoter in which a part of the LTR is replaced with other promoter may also be utilized.

When the introduction factor is a gene expression product (e.g., protein), a peptide called Protein Transduction Domain (PTD) or the like is bound to a protein as an expression product and added to the medium to introduce the gene expression product into somatic cells.

Culture

In the method of the present invention, differentiated somatic cells of a mammal can be cultured in a medium after introduction of a gene. For example, it is a preferred embodiment when inducing (generating) urothelial cells in vitro.

Culturing can be performed in a suitable container for storing cells and media. As a method for performing suitable culture, a method of culturing under conditions of about 37°C and a carbon dioxide concentration of about 5% is exemplified, but the method is not limited thereto. Culturing under the above-mentioned conditions can be performed using, for example, a known $CO_2$ incubator.

The period for culturing is not particularly limited as long as the effect of the present invention is not impaired. For example, it can be about 12 hr to 1 month, about 1 day to 3 weeks, or about 3 days to 2 weeks. If necessary, the medium can be exchanged. The culture conditions are preferably those of a conventional method.

In culture, passage can be performed if necessary. When passage is performed, the cells are collected before or immediately after reaching a confluent state and seeded in a fresh medium. In the culture of the present invention, moreover, the medium can also be replaced as appropriate.

Medium

A medium to be used in the method of the present invention is not particularly limited. General liquid media such as MEM (Modified Minimum Essential medium), DMEM (Dulbecco's Modified Eagle's medium), EMEM (Eagle's minimal essential medium), αMEM (alpha Modified Minimum Essential medium) and the like can be used. Where necessary, components such as serum components (Fetal Bovine Serum (FBS), Human Serum (HS)), antibiotics such as streptomycin, penicillin and the like, non-essential amino acid (NEAA), and the like can be added.

From the aspect of high efficiency in generating urothelial cells by the method of the present invention, it is preferable to use, as a medium, a differentiation-inducing medium generally used for differentiating urothelial cells. The "differentiation-inducing medium for differentiating urothelial cells" refers to a medium containing a component capable of differentiating pluripotent stem cells (ES cell, iPS cell, and the like) into urothelial cells (hereinafter to be also referred to as the urothelial cell induction medium of the present invention).

As the urothelial cell induction medium, a known medium can be used. For example, the media described in Osborn S.L. et al., Stem Cells Transl Med. 2014; 3(5): 610-619. and Kang M. et al., Int. J. Mol. Sci. 2014, 15(5), 7139-7157 can be used. However, the medium is not limited to these.

Preferably, the urothelial cell induction medium of the present invention contains 3-isobutyl 1-methylxanthine (IBMX) and epidermal growth factor (EGF). The concentration of IBMX in the medium is generally 1 μM - 10 mM; however, it is preferable that the medium contains 1 mM - 10 mM IBMX for selective maintenance and proliferation of urothelial cells. The concentration of EGF in the medium is generally 0.001 - 1 ng/mL, preferably 0.01 - 1 ng/mL. When these components are contained, the conversion from fibroblasts to urothelial cells and subsequent selective maintenance and proliferation of the urothelial cells become more efficient, as shown in the Examples.

One preferred example of the urothelial cell induction medium of the present invention is a medium having the following composition (to be also referred to as "UCM medium" in the present specification).

EpiLife™ medium supplemented with

60 μM calcium,
60 μg/mL bovine brain hypophysis extract,
5 μg/mL human gene recombinant insulin,
gentamicin amphotericin,
2% FBS,
0.01 ng/mL human gene recombinant EGF,
1 mM IBMX, and
1 μM tranylcypromine.

(EpiLife™ medium: medium for culturing keratinocytes)

Induction (generation)

As a result, urothelial cells are induced from somatic cells. The formation of urothelial cells can be detected by, for example, gene expression of Uroplakin (Uroplakinlb; UPK1), Uroplakin2 (UPK2), CK5, CK17, formation of Asymmetric Unit Membrane, and the like.

The induced urothelial cell contains, as exogeneous factor, at least one, preferably two, more preferably three, and even more preferably all four members selected from the group consisting of FOXA1 gene or an expression product thereof, TP63 gene or an expression product thereof, MYCL gene or an expression product thereof, and KLF4 gene or an expression product thereof. In another embodiment, the exogenous factor contains FOXA1 gene or an expression product thereof, and TP63 gene or an expression product thereof, depending on the type and number of genes to be introduced. In yet another embodiment, it contains the following genes or expression products thereof, or a combination thereof.

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

As used herein, "exogeneous" mainly refers to an embodiment of a gene or an expression product thereof introduced as a result of the above-mentioned introduction means, which is different from natural embodiments. For example, a gene whose expression is controlled by a promoter other than natural promoters, a position on a chromosome other than natural ones, an embodiment of a gene existing outside the chromosome, and the like can be mentioned.

Urothelial cells may be obtained as a mixture with cells other than the urothelial cells (e.g., the original somatic cells). In such cases, the urothelial cells and the cells other than the urothelial cells can be separated where necessary. The means for separation is not particularly limited. For example, they can be separated using a cell sorter or magnetic beads.

The urothelial cells induced by the present invention can be preferably used, for example, as a transplant material.

The urothelial cells induced by the present invention can also be used for various studies, technical developments, and the like using urothelial cells. For example, they are useful for basic researches such as analyses of the mechanism of the development, differentiation, and morphogenesis of the urinary organs, and the influence of mechanical stress, nutrition, hormone, and the like on them.

By using the urothelial cell induced by the present invention, urothelial cells can be easily, quickly, and inexpensively established from human and animals having various diseases and genetic backgrounds. Thus, abnormalities of urothelial cells that are related to diseases and genetic backgrounds can be analyzed by biochemical, molecular biological, or immunological methods, and the like, which will be useful for researches such as elucidation of the onset mechanism of diseases and the development of diagnostic methods. Furthermore, the development of medicaments, toxicity test of medicaments, and the like using such urothelial cells are useful for the development of a new therapeutic method for various diseases.

The urothelial cells obtained by the present invention can be used for treating various diseases, particularly urologic diseases. For example, the urothelial cells induced by the present invention can be used in the formation of a substitute bladder in a case of total cystectomy for bladder cancer, the construction of a patch to the site where the urothelium is defective in bladder-vaginal fistula, formation of urothelium for partial supplement in urinary bladder enlargement surgery for cases of bladder fibrosis and atrophy due to neurogenic bladder, urothelial regeneration for severe interstitial cystitis (dysfunction of urothelium), and the like.

Specific diseases to be treated include bladder cancer, overactive bladder, congenital bladder abnormality (e.g., bladder exstrophy, bladder diverticulum, urinary diverticulum abnormality), bladder injury, interstitial cystitis (Hunner type and non-Hunner type), neurogenic bladder, contracted bladder, and the like.

In the present specification, unless otherwise indicated, the term "treatment" is intended to mean an action to be performed while a patient is suffering from a particular disease or disorder, whereby the severity of the disease or disorder, or one or more symptoms thereof are alleviated or the progression of the disease or disorder is delayed or slowed down. In the present specification, the "treatment" includes "prophylaxis".

The urothelial cells obtained by the present invention can also be used not only for the treatment of diseases but also for the purpose of cosmetology and functional enhancement. The action for humans in that case is also referred to as a treatment in the present specification for convenience, and "patient" can be read as "healthy person" or "human", and "disease" can be read as "cosmetology" or "function".

The present invention can also be used for the treatment of diseases of not only humans but also pet animals such as dog, cat, and the like and mammals including domestic animals such as cow, horse, pig, sheep, chicken, and the like. In this case, "patient" is read as "animal patient" or "mammal".

Composition

As described above, urothelial cells can be induced by introducing, as exogeneous factor, at least one, preferably two, more preferably three, and even more preferably all four members selected from the group consisting of FOXA1 gene or an expression product thereof, TP63 gene or an expression product thereof, MYCL gene or an expression product thereof, and KLF4 gene or an expression product thereof into somatic cells, Therefore, the present invention further provides a composition for inducing urothelial cells, containing at least one, preferably two, more preferably three, and even more preferably all four members selected from the group consisting of FOXA1 gene or an expression product thereof, TP63 gene or an expression product thereof, MYCL gene or an expression product thereof, and KLF4 gene or an expression product thereof. The composition for inducing urothelial cells contains a factor used for inducing urothelial cell from somatic cell, and it is desirable that at least one, preferably two, more preferably three, and even more preferably all four members selected from the group consisting of FOXA1 gene or an expression product thereof, TP63 gene or an expression product thereof, MYCL gene or an expression product thereof, and KLF4 gene or an expression product thereof are contained in a form permitting introduction into somatic cells. In another embodiment, the exogenous factor contains FOXA1 gene or an expression product thereof, and TP63 gene or an expression product thereof, depending on the type and number of genes to be introduced. In yet another embodiment, it contains the following genes or expression products thereof, or a combination thereof.

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

Specific examples of the form that enables introduction of the above-mentioned genes into somatic cells include a vector incorporating the above-mentioned genes. Here, the above-mentioned genes may be incorporated in different vectors, or two or more kinds of genes may be simultaneously incorporated in one vector.

The type of vector that can be used, and the like are as described above.

The above-mentioned composition can be used, for example, as a medicament (therapeutic drug) in gene therapy.

Direct reprogramming in vivo

By introducing at least one, preferably two, more preferably three, and even more preferably all four members selected from the group consisting of FOXA1 gene or an expression product thereof, TP63 gene or an expression product thereof, MYCL gene or an expression product thereof, and KLF4 gene or an expression product thereof into somatic cell (e.g., fibroblast) present at the site where urothelial cell is defective, urothelial cells can be induced at the damaged site by direct reprogramming, thereby contributing to the treatment of urothelial cells and the regeneration of urothelial cells. For introduction of the FOXA1 gene or an expression product thereof, TP63 gene or an expression product thereof, MYCL gene or an expression product thereof, and KLF4 gene or an expression product thereof, the above-mentioned composition of the present invention can be preferably used.

It is understood that the induction method of the present invention includes the above-mentioned in vivo direct reprogramming in addition to in vitro direct reprogramming. Such in vivo direct reprogramming enables, for example, gene therapy to treat various diseases. Specific examples of the disease include those described above.

The in vivo direct reprogramming can be performed according to the direct reprogramming to cardiomyocytes in vivo described in, for example, the following documents except that at least one, preferably two, more preferably three, and even more preferably all four members selected from the group consisting of FOXA1 gene or an expression product thereof, TP63 gene or an expression product thereof, MYCL gene or an expression product thereof, and KLF4 gene or an expression product thereof are introduced into somatic cells (e.g., fibroblast) in the damaged area of urothelial cells.

1: Ieda M. Heart regeneration using reprogramming technology. Proc Jpn Acad Ser B Phys Biol Sci. 2013; 89(3):118-28. Review.
2: Ieda M, Fu JD, Delgado-Olguin P, Vedantham V, Hayashi Y, Bruneau BG, Srivastava D. Direct reprogramming of fibroblasts into functional cardiomyocytes by defined factors. Cell. 2010 Aug 6; 142 (3) :375-86.
3: Qian L, Huang Y, Spencer CI, Foley A, Vedantham V, Liu L, Conway SJ, Fu JD, Srivastava D. In vivo reprogramming of murine cardiac fibroblasts into induced cardiomyocytes. Nature. 2012 May 31; 485(7400):593-8.

In addition, it can also be performed injecting at least one, preferably two, more preferably three, and even more preferably all four members selected from the group consisting of FOXA1 gene or an expression product thereof, TP63 gene or an expression product thereof, MYCL gene or an expression product thereof, and KLF4 gene or an expression product thereof into the lumen such as bladder, renal pelvis, and the like. The above-mentioned gene or an expression product thereof or a combination thereof is injected into the bladder or the renal pelvis with a urethral catheter or the like, and the somatic cells present in the bladder or the renal pelvis are brought into contact with the gene or an expression product thereof or a combination thereof, whereby the somatic cells are induced into urothelial cells.

The contents of the above-mentioned documents 1 - 3 are incorporated herein by reference.

[Example]

While Examples are shown in the following, the present invention is not limited to the Examples alone.

<<List of Abbreviations>>

aHDF (adult Human Dermal Fibroblast; adult human skin fibroblast)
UC (urothelial cell)
HUC (Human urothelial cell)
HUVEC (Human Umbilical Vein Endothelial Cell)
dUC (directly converted UC)
FOXA1 (Forkhead box A1; to be also referred to as "F" in the present specification)
TP63 (tumor protein P63; to be also referred to as "T" in the present specification)
MYCL (L-Myc; to be also referred to as "L" in the present specification)
KLF4 (Kruppel-like factor 4; to be also referred to as "K" in the present specification)
IRF1 (Interferon regulatory factor 1; to be also referred to as "I" in the present specification)
GFP (green fluorescent protein)

«Materials and Methods»

1. Cell culture

aHDFs were seeded on a laminin-coated 12-well plate or a laminin-uncoated 12-well plate, and the gene was introduced using a retroviral vector. The cells were cultured in CnT-Prime medium or other medium, and 21 days later, observation with a phase contrast microscope, real-time PCR, and analysis of immunocytochemistry were performed. The cells were passaged in fresh culture plates and used for RNA sequencing and penetration assay. In the in vivo experiment, cells into which F, T, L, and K had been introduced were transurethrally transplanted into the injured bladder cavity of an interstitial cystitis mouse through a catheter.

2. Medium

10% FBS (Gibco)-added high-glucose DMEM supplemented with MEM non-essential amino acid, 100 mM sodium pyruvate, and 100 U/mL penicillin-streptomycin was used as "Standard Medium".

"CnT-Prime (registered trade mark)" medium was purchased from CeLLnTEC.

"Uromedium" medium was produced according to the method of Osborn et al. (Osborn SL, (2014) Stem Cell Transl Med 3(5):610-619.). In summary fashion, the "Uromedium" medium is composed of EpiLife™ medium supplemented with 60 μM calcium (Gibco; MEP1500CA), 60 μg/mL bovine brain hypophysis extract (Gibco), 5 μg/mL human gene recombinant insulin (Diagnocine), 500 ng/mL hydrocortisone (Tokyo Chemical Industry Co., Ltd.), gentamicin·amphotericin (Gibco), 2% FBS, 0.1 ng/mL human gene recombinant epidermal growth factor (EGF) (RSD), and 100 μM 3-isobutyl 1-methylxanthine (IBMX) (Sigma-Aldrich).

The "UCM" medium is composed of EpiLife™ medium supplemented with 60 μM calcium, 60 μg/mL bovine brain hypophysis extract, 5 μg/mL human gene recombinant insulin, gentamicin·amphotericin, 2% FBS, 0.01 ng/mL human gene recombinant EGF, 1 mM IBMX, and 1 μM tranylcypromine (Abcam).

3. Cells

aHDFs and Plat-GP cells were purchased from ScienCell Research Laboratories and Cell Biolabs, respectively, and cultured in Standard Medium. aHDFs at passage number of not more than 10 were used for experiments. HUCs were purchased from KURABO INDUSTRIES LTD. (KP-4309; Lot04101). Induced pluripotent stem cells (iPS cells) were purchased from JCRB cell Bank, and maintained according to the previous report (Nakagawa M, et al., (2014) Sci Rep 4(3594):10.1038/srep03594.1). In summary fashion, iPS cells were cultured on a plate coated with Easy iMatrix-511 silk (Nippi, Incorporated; 0.25 $\mu g/cm^2$; treated in advance at 37°C for 1 hr) in a StemFit medium (Ajinomoto Co., Inc.; AK02N). For passage, iPS cells were dissociated into a single cell suspension in StemFit medium containing Rock inhibitor (Nacalai Tesque; Y-27632), and reseeded in new culture plates. The next day, the medium was replaced with fresh StemFit medium without Rock inhibitor, and the medium was changed every other day. Passage was performed when iPS cells reached 80% to 90% confluency. T24, human bladder carcinoma cell line, was provided by professor Ashihara (Kyoto Pharmaceutical University, Kyoto, Japan), and cultured in RPMI1640 medium supplemented with 10% FBS, MEM non-essential amino acid, 100 mM sodium pyruvate and 100 U/mL penicillin-streptomycin.

4. Antibody

As primary antibodies for immune antibody staining, rabbit polyclonal anti-uroplakin 1b IgG (Abcam; #ab102961; dilution 1:100), rabbit polyclonal anti-uroplakin 2 IgG (Proteintech; #21149-1-AP; 1:100), mouse monoclonal anti-E-cadherin IgG (BD Biosciences; #610181; 1:50), rabbit polyclonal anti-Nanog IgG (Reprocell; #09-0020; 1:200), and mouse monoclonal anti-keratin 8/18 IgG (CST Japan; #4546S; 1:50), and rabbit monoclonal anti-keratin 20 IgG (CST Japan; #13063; 1:100) antibodies were used. As secondary antibodies, the following antibodies were used. goat Alexa Fluor 488-conjugated anti-rabbit IgG and anti-mouse IgG antibodies (Life technologies; #A11034 and #11029; 1:500), and goat Alexa Fluor 594-conjugated anti-rabbit IgG and anti-mouse IgG antibodies (Life technologies; #A11037 and #11032; 1:500).

5. Retroviral vector

Full length cDNA clones for FOXA1, IRF1, TP63, and Sonic hedgehog genes were purchased from DNAFORM library, and coding sequences were amplified by RT-PCR with KOD-Plus-Neo DNA polymerase (TOYOBO CO., LTD.). The primer sequences were as follows.

FOXA1

5’-CAGCAGTGTGGTGGTACGGGATGTTAGGAACTGTGAAGATG-3’ (SEQ ID NO: 1)
5’-ACCGGCGCTCAGCTGGCTAGGAAGTGTTTAGGACGGG-3’ (SEQ ID NO: 2)

IRF1

5’-CAGTGTGGTGGTACGGGATGCCCATCACTCGGATGCGC-3’ (SEQ ID NO: 3)
5’-ACCGGCGCTCAGCTGGACCGGCGCTCAGCTGG-3’ (SEQ ID NO: 4)

TP63

5'-CAGTGTGGTGGTACGGGATGAATTTTGAAACTTCACGG-3' (SEQ ID NO: 5)
5'-ACCGGCGCTCAGCTGGCTATCACTCCCCCTCCTCTTT-3' (SEQ ID NO: 6)

sonic hedgehog

5'-CAGTGTGGTGGTACGGGATGCTGCTGCTGGCGAGATGT-3' (SEQ ID NO: 7)
5'-ACCGGC GCTCAGCTGGCTAGCTGGACTTGACCGCCAT-3' (SEQ ID NO: 8)

The amplified DNA fragments were cloned into PMxs retroviral plasmid vector (Cell Biolabs) using the Gene Art System (Invitrogen). pMxs plasmids containing human POU5F1, KLF4, MYCL, GFP genes were provided by professor Yamanaka (CiRA, Kyoto University, Kyoto, Japan). Plat-GP cells were seeded in 100 mm plates at a density of $3\times10^6$ cells/dish and cultured for 24 hr. The aforementioned 5.0 μg of pMxs and 2.5 μg of pCMV-VSV-G were introduced into the cells by using XtremeGENE 9 DNA Transfection Reagent (Sigma-Aldrich). After 24 hours the medium was replaced with antibiotic-free Standard Medium. The supernatant of the culture medium was cultured and used as a retroviral suspension.

6. Conversion of fibroblasts into dUCs

aHDFs were seeded in 12-well plates coated with atelocollagen (KOKEN; I-PC30), poly-L-lysine (ScienCell), or laminin (Easy iMatrix-511 silk) at a density of $3\times10^4$ cells/well. After 24 hr, the medium was replaced with the retroviral suspension that was filtrated through a 0.45 μm pore filter (Toyo Roshi Kaisha, Ltd., ADVANTEC) and supplemented with 4 μg/mL polybrene. The next day, the culture supernatant was replaced with Standard Medium free of virus, and replaced with CnT-Prime or "UCM" medium 3 days later. The culture medium was changed every 3 or 4 days.

7. Passages of dUCs

dUCs were passaged when they reached 60 to 80% confluency or 14 days after the previous passage. dUCs were detached using trypsin/EDTA (Nacalai Tesque) and Cell Scraper (IWAKI), reseeded in laminin-coated 12-well plates at a density of $1\times10^4$ cells/well, and cultured in "UCM" medium.

8. Differentiation induction of iPS cell into urothelial cell

iPS cells were converted to urothelial cells by the method of the previous report (Osborn SL, et al., (2014) Stem Cell Transl Med 3(5):610-619.) after slight modification. In summary fashion, iPS cells were seeded in laminin-coated 6-well plates at a density of $2\times10^4$ cells/well and cultured until they reached 60 to 70% confluency. The medium was replaced with RPMI1640 supplemented with 2% FBS, GlutaMAX (Gibco), 100 U/mL penicillin-streptomycin, and 5 μM IDE1 (Cayman). The medium was replaced with a fresh one every 1-2 days. Nine days after differentiation induction into definitive endoderm (DE) cells, the medium was replaced with "Uromedium" to allow differentiation of the DE cells into urothelial cells. The medium was changed every 2-3 days during the 18 day culture period, and the cells were cultured in a medium containing 10 mM Rosiglitazone (Sigma-Aldrich) and not containing EGF for 3 days.

9. Real-time RT-PCR

Total RNA was extracted using RNeasy Mini Kit (Qigen) according to the manufacturer's protocol. Total RNA of human urothelial cell (HUC) was purchased from ScienCell Research Laboratories (#4325, Lot 4740). Total RNAs of human small intestine, liver were purchased from Clontech Laboratories (#636539, Lot 1611206A; #636531, Lot 1703002). Total RNA of human salivary gland was purchased from BioChain (R1234212-10, Lot B111155). Total RNA of endothelial cell was extracted from human umbilical vein endothelial cell (HUVEC; PromoCell; C-12200). cDNA was synthesized using ReverTra Ace qPCR (TOYOBO Life Science), and subjected to real-time RT-PCR using StepOnePlus Real-Time PCR Systems (Applied Biosystems). The reaction mixture included Taqman probe (Applied Biosystems) and Taqman Fast Advanced Master Mix (Applied Biosystems). The relative mRNA expression levels were calculated from the following formula:

Relative mRNA level (fold) = [(target gene mRNA level in sample/β-actin gene mRNA level in sample)]/[(target gene mRNA level in control sample)/(β-actin gene mRNA level in control sample)].

10. Immunocytochemical analysis

Cells cultured in 12-well plates were washed with PBS(-), fixed with 4% paraformaldehyde (PFA) for 30 min, and subjected to a membrane permeation treatment using 0.2% Triton-X 100 (Nacalai Tesque) at room temperature for 15 min. Background was blocked by adding Blocking One Histo (Nacalai Tesque) for 30 min at room temperature, and the cells were reacted with a primary antibody overnight at 4°C. After washing, secondary antibodies were added and the cells were reacted for 1 h at room temperature in the dark. The cells were washed and cell nuclei were stained with Hoechst 33342 (DOJINDO) for 5 min. The cells were observed by BZ-X (Keyence). Positive or negative cells for each signal were counted by BZ-X Analyzer software (Keyence). The percentage of signal-positive cells was calculated from the following formula.

% of signal positive cell =(signal positive and Hoechst positive cells/total number of Hoechst 33342 positive cells)x 100

11. RNA sequence

Total RNA was extracted from aHDFs and P2 dUCs (cultured in UCM) with the RNeasy Mini Kit (QIAGEN) according to the manufacturer's protocol. Total RNA from human urothelial cells (HUCs) was purchased from ScienCell Research Laboratories. The quality of each RNA sample was checked by agarose gel electrophoresis (gel concentration: 1%, voltage: 180 V, electrophoresis time: 16 min) and Agilent 2100 analysis. Library preparation and sequencing were performed by Novogen Inc. Briefly, ribosome RNA was removed using the Ribo-Zero kit. The mRNA was randomly fragmented by adding a fragmentation buffer, and cDNA was synthesized. The quality-checked libraries were sequenced with Illumina Novaseq 6000 (Illumina; paired-end, 150 bp). Filtered sequence data were mapped on Reference genome (GRCh38) using HISAT2 version 2.1.0 (https://ccb.jhu.edu/software/hisat2/index.shtml). After removing residual ribosome RNA from the sequence results identified by Bedtools (https://bedtools.readthedocs.io/en/latest/), the mapped sequences were counted using featurecounts (http://bioinf.wehi.edu.au/featureCounts/) (Liao, Y., Smyth, G. K. & Shi, W. featureCounts: an efficient general purpose program for assigning sequence reads to genomic features. Bioinformatics 30, 923-930 (2014)), and associated with the annotation GTF file from GENCODE (GENCODE 29; https://www.gencodegenes.org/). Analysis was performed using iDEP8.1 (Ge, S. X., Son, E. W. & Yao, R. iDEP: an integrated web application for differential expression and pathway analysis of RNA-Seq data. BMC Bioinformatics 19, 10.1186/s12859-018-2486-6 (2018)). The read count data were normalized by CPM (counts per million) function in edgeR, and the genes that did not have not less than 0.5 counts/million in at least one sample were eliminated. Converted data were used for search analysis (hierarchical clustering analysis and gene expression variation analysis), and non-converted data were used for pathway analysis by GSEA (Gene Set Enrichment Analysis) as fold-change values returned by DESeq2. The gene expression data was visualized on KEGG pathway diagrams (Kanehisa, M., Furumichi, M., Tanabe, M., Sato, Y. & Morishima, K. KEGG: new perspectives on genomes, pathways, diseases and drugs. Nucleic Acids Res 45, D353-D361 (2017)) using Pathview (Luo, W. & Brouwer, C. Pathview: an R/Bioconductor package for pathway-based data integration and visualization. Bioinformatics 29, 1830-1831 (2013)).

12. In vitro permeability assay

HTS Transwell clear 12-well plates were purchased from Corning Incorporated, and the inner chambers thereof, which contained polycarbonate membrane with a diameter of 12 mm with 0.4 μm pores, were coated with Easy iMatrix-511 silk (Nippi, Incorporated; 0.25 $\mu g/cm^2$, preincubated at 37°C for 1 hr). P4 dUCs or aHDFs were resuspended in UCM medium and seeded in the inner chamber at a density of $1\times10^5$ cells/well. HUCs were resuspended in Urolife™ D Complete Medium (Urolife) and seeded in the inner chamber at a density of $1\times10^5$ cells/well. For final differentiation, a part of HUCs were cultured in Urolife added with 1 μM Rosiglitazone (PPARγ activator; Sigma-Aldrich) and 1 μM

PD153035 (EGFR inhibitor; TCI) for 4 days. The cells were cultured for 5 days and then washed with PBS(-). FITC-labeled dextran with a molecular weight of about 4 kDa (IWAI CHEMICALS COMPANY LTD.) was added into the inner chamber at a concentration of 2 mg/mL. After culturing incubation for 20 min under shading, the supernatant in the outer chamber was transferred into 96-well plates, and fluorescence intensity in each well was measured by SpectraMax M2 (Molecular Devices) with fluorescence 533nm, excitation 485 nm. The relative value (%) of the FITC-labeled dextran leaked into the outer chamber to that of the control without seeding of cells as 100% was calculated by the following formula:

%=(fluorescence intensity in each well/fluorescence intensity without addition of FITC-labeled dextran)/(fluorescence intensity in control well/fluorescence intensity without addition of FITC-labeled dextran) x 100

13. Transplantation of dUCs into mouse bladder

All animal experiments were approved by the Committee for Animal Experiments of the university (M30-281). The handling of the animals was in accordance with the guideline of the university. Female NOG/BALB-Rag2nullIL-2Rγnull/NSG (NOG/SCID) (NOG/SCID) mice were purchased from CLEA Japan, Inc. An interstitial cystitis model of mice and intra-bladder cell transplantation method were as in the previous report (Homan T, et al., (2013) J Pharmacol Sci 121(4):327-337., Shimizu T, et al., (2018) Oncoimmunology 7(5) :e1424671.) with slight modifications. 6- to 8-week-old mice under anesthesia were intraperitoneally injected with anti-asialo GM1 antibody at a dose of 100 mg/mouse. At the same time, a 24-gauge catheter was inserted into the bladder through the urethra, and 50 μL of 1.5% $H_2O_2$ solution was injected into the bladder. One hour later, the bladder was washed with PBS(-), and a solution containing 50 μL of 12,000 PU dispase II (FUJIFILM Wako Pure Chemical Corporation) was injected into the bladder, and Easy iMatrix 511 silk was administered for 1 hr. aHDFs with introduction of F, T, L, and K thereinto were cultured in Standard Medium for 4 days, suspended in PBS(-) at a concentration of $3\times10^6$/100 μL, and transplanted into the bladder (n=4 each). In other groups, aHDFs ($3\times10^6$ cells/100 μL PBS) free of gene introduction or PBS(-) alone was injected into the bladder. The urethra was sutured, and the suture was removed 3 hr later to allow spontaneous urination.

14. Study of tumorigenicity in vitro

6- to 8-week-old female NOG/SCID mice were anesthetized and 100 mg of anti-asialo GM1 antibody was intraperitoneally injected. aHDFs with introduction of F, T, L, and K thereinto were cultured in Standard Medium for 4 days, mixed with Matrigel (Corning), and subcutaneously administered to the mice at a concentration of $1\times10^7$/100 μL. T24 cells and aHDFs free of gene introduction were used as a positive control and negative control, respectively. The diameter of the tissue swelling at the administration site, or when a tumor was formed, the diameter of the tumor was measured with a caliper every week for 10 weeks, and the volume was calculated.

15. Immunohistochemistry

The bladder was removed from the mice 7 days after the cell transplantation. After fixation in formaldehyde, in some organs, it was substituted with 30% sucrose/PBS(-). Other parts were embedded in paraffin. Samples were sliced into 5-10 μm thickness. After blocking the endogenous peroxidase activity with 3% $H_2O_2$ for 5 min, they were incubated with Protein Block (Dako) for 30 min. After washing, tissue sections were reacted with primary antibodies overnight at 4°C. The tissues were washed and reacted with Alexa Fluor 594-labeled goat anti-rabbit IgG or anti-mouse IgG secondary antibodies for 60 min at room temperature. After washing 3 times, tissue sections were encapsulate using VECTASHIELD mounting medium containing DAPI (Vector Laboratories). Fluorescence was observed using BZ-X (Keyence).

16. Statistical analysis

Each data is expressed in mean±SD. Statistical significance was tested by Student's t-test or one-way ANOVA and post hoc Tukey-Kramer test. The statistically significant difference was set at $P<0.05$. All analyzes were performed using EZR (Kanda, Y. Investigation of the freely available easy-touse software 'EZR' for medical statistics. Bone. Marrow. Transplant. 48, 452-458 (2013)).

Example 1

Adult human skin fibroblasts (aHDFs) were induced to express urothelial cell-like phenotype by introducing specific factor.

First, three transcription factors were focused on. FOXA1(F) and IRF1(I) are important in the development of urothelial cells. On the other hand, TP63 (T) is a transcription factor that is expressed in epithelial stem cells and is associated with the development and differentiation of urothelial cells. In addition, sonic hedgehog (SHH) gene (H), which is expressed in the basement membrane cells of urothelial cells and regulates signals important for cell proliferation during the regeneration process, was added. These genes were introduced into adult human skin fibroblasts (aHDFs) using a retroviral vector and the cells were cultured in CnT-Prime medium for a period considered to be suitable for cell maintenance (21 days) (Fig. 1). In cells into which FIT or FIH was introduced, uroprakin (UPK1b), which is a development marker for urothelial cells, was slightly expressed (Fig. 2A). Neither cell formed urothelial cell-like colonies (Fig. 2B). Then, three reprogramming genes, POU5F1 (P), KLF4 (K), and MYCL (L), were added. High levels of UPK1b mRNA were expressed in some cells in which genes associated with urothelial cells and reprogramming genes were combined. This suggests that inclusion of FOXA1 in the gene combination is essential (Fig. 3). Then, the need for I, T and H genes was confirmed. As a result, it was found that T or H, not I, promote epithelial cell colony formation (Fig. 4). The need for P, K, and L was determined by combining with FT. The combination of FTLK induced mRNA expression of UPK1b and formation of epithelial cell colony (Figs. 5 A, B). In addition, FTLK-introduced cells expressed uroplakin 2 (UPK2) mRNA that is another urotheliumspecific marker (Fig. 5 A). While UPK1b is expressed not only in urothelial cells but also in corneal epithelium and conjunctival epithelium, UPK2 has been reported to have stronger specificity to urothelial cells (Habuka, M. et al., PLoS. One. 10, e0145301 (2015)).

Whether F, T, L, and K are necessary and sufficient for inducing the expression of UPK1b and UPK2 was verified. The mRNA expression level of UPK1b increased in cells converted with F, L, and K, and the mRNA expression level of UPK2 was very high in the combination of T, L, and K. The combination of L and K and the combination of F, L, and K also significantly increased the mRNA expression level of UPK2. The combination of F, T, L, and K induced increased mRNA expression in both UPK1b and UPK2 (Fig. 6 A). From these results, it was concluded that F, T, L, K is the optimal gene combination that induce the phenotype of urothelial cell in aHDF.

How the coating of the culture plates affects the efficiency of cell conversion was examined. Before seeding aHDFs, the culture plates were coated with collagen, poly-L-lysine, or laminin. Epithelial cell colonies were formed only in laminin-coated plates (Fig. 6B), and the expression of UPK1b and UPK2 mRNAs was the highest (Fig. 6C).

Cells with or without introduction of F, T, L, and K thereinto were cultured in various media. As shown in Figs. 6 D, E and Fig. 7, the cells converted by F, T, L, and K formed urothelial cell colonies best in CnT-Prime medium and showed remarkably high mRNA expression levels of UPK1b and UPK2. The "Uromedium" medium, which is often reported to be suitable for culturing urothelial cells, has been found to support not only the formation of epithelial cell colonies but also the proliferation of fibroblast-like cells. These results suggest that CnT-Prime medium is superior in the conversion to urothelial cells. In contrast, conversion of cells without gene introduction to urothelial cells did not occur in CnT-Prime medium.

Example 2

Characteristics of directly-converted urothelial cells (dUCs).

The cells transduced by the method of Example 1 are indicated as directly-converted urothelial cells (dUC). Then, the detailed characteristics of the cells were investigated. In the time-course analysis, cells transduced by F, T, L, and K markedly expressed UPK1b 8 days after gene introduction, and the expression peaked 15 days later (Fig. 8a). The UPK2 mRNA expression began to rise after 11 days in the FTLK-transduced cells and peaked 21 days later. The formation of epithelial cell colony was observed after 11 days and increased thereafter (Fig. 8b).

In addition to the expression of UPK1b and UPK2, the expression of E-cadherin, which is a transmembrane type protein expressed in general epithelial cells, and keratin 8/18, which is a cytoskeleton, was confirmed. Immunocytochemical analysis revealed that about 42% of the cells expressed UPK1b, whereas 23%, 26%, and 26% of UPK2-positive, E-cadherin-positive, and keratin 8/12-positive cells respectively expressed UPK1b (Fig. 8c, d). This result suggests that the conversion rate was about 25%.

Osborn et al. reported a method for differentiating iPS cells into urothelial cells via endoderm (Osborn SL, et al., (2014) Stem Cell Transl Med 3(5) :610-619.) . Therefore, dUCs prepared by direct conversion were compared with iPS cellderived urothelial cells (iUCs) induced by the method of Osborne et al. after slight modification (Fig. 9 A). dUCs strongly expressed the UPK1b and UPK2 genes, whereas iUCs strongly expressed the UPK1a and UPK3a genes (Fig. 9B). This strongly suggests that dUCs resemble immature urothelial cells more closely than iUCs.

Example 3

Pluripotency, liver, small intestine markers were not expressed in dUCs.

To exclude the possibility that fibroblasts were temporarily converted to iPS cells or endodermal progenitor cells that subsequently produced urothelial cells, expression of pluripotent markers and non-urothelial endodermal markers, and the expression of non-urothelial endodermal markers in cells into which F, T, L, and K were introduced during the conversion process were examined. As a result, gene expressions of NANOG, POU5F1, LIN28, and SOX2 were not observed during the culture period in cells into which F, T, L, and K were introduced. NANOG protein was not observed, either (Fig. 10). Similarly, cells into which F, T, L, and K had been introduced did not express mRNA of small intestinal epithelial cell marker CDX2 and hepatocyte marker albumin (ALB) at any observation time (Fig. 11). In addition, cells into which F, T, L, and K had been introduced did not express mRNA of CD31, which is a mesenchymal-derived endothelial cell marker, or AQP5, which is a ectoderm-derived salivary gland marker (Fig. 11). Therefore, it was confirmed that cells into which F, T, L, and K had been introduced did not undergo the pluripotent stage in the process of conversion to dUCs, and did not convert to cells derived from other endoderms. It was also confirmed that they did not convert to mesenchymal or ectoderm cells.

Example 4

dUCs can be selectively proliferated by passage culture.

The dUC colonies formed in 21 days after gene introduction did not grow actively even when continuously cultured on the same medium. To secure a large amount of dUCs with high purity, cell culture was started in the first medium (generation number 0, P0) and then passaged in a new medium (generation number 1, P1). However, when P0 dUCs prepared in the CnT-Prime medium were newly cultured in the same medium, almost no epithelial cell colonies were formed, and only a few non-epithelial cells proliferated. The "Uromedium" did not form a pavingstone-like colony even in the next generation (Fig. 12). To provide a medium suitable for dUCs, therefore, the concentration of the medium components contained in "Uromedium" was modified and the composition was determined.

As shown in Fig. 13A, the concentrations of insulin and hydrocortisone were not definitive. On the other hand, 1-10 mM IBMX played a beneficial role, including expression of UPK2 mRNA by cells into which F, L, T, and K were introduced . Both hydrocortisone-free medium and medium containing 10 mM IBMX allowed cells into which F, L, T, and K were introduced to form epithelial cell colonies (Fig. 13B). EGF was reported to be not required for proliferation and differentiation of urothelial cells. However, addition of 0.01-1 ng/mL EGF increased mRNA expression of UPK2 (Fig. 13A). Addition of tranylcypromine, an irreversible monoamine oxidase inhibitor known to increase the efficiency of reprogramming from iPS cells into fibroblasts, also enhanced formation of urothelial cell colonies (Fig. 13C).

Based on these results, the most suitable composition of culture medium for the passage of dUCs was determined. This medium named urothelial cell conversion maintenance medium (UCM) has the following composition.

EpiLife™ medium supplemented with

60 μM calcium,
60 μg/mL bovine brain hypophysis extract,
5 μg/mL human gene recombinant insulin,
30 μg/mL gentamicin,
15 ng/mL amphotericin,
2% FBS,
0.01 ng/mL human gene recombinant EGF,
1 mM IBMX, and
1 μM tranylcypromine.

(EpiLife™ medium: medium for keratinocyte culture)

UCM supported epithelial cell colony formation of cells into which F, L, T, and K were introduced and suppressed the growth of fibroblast-like cells (Fig. 12, Fig. 13D). Then, whether this characteristic medium can be used to support passage culture and selective growth of dUCs was investigated (Fig. 14a). As shown in Fig. 15a, at least 4-generation passage culture of dUCs was successful. At P4, dUCs strongly expressed UPK1b, UPK2, and E-cadherin (Figs. 15b, c and Figs. 14b, c). These results suggest that, in UCM medium, dUCs proliferated more efficiently than fibroblast-like cells and stably remained in urothelial-like cells during the passage process.

Example 5

dUCs exhibit remarkable barrier function in vitro.

Then, the barrier function of P4 dUCs was checked using an in vitro permeability assay using a transwell. P4 dUCs were cultured to confluence on the bottom membrane of a laminin-coated inner chamber (Fig. 15d). Thereafter, FITC-labeled dextran (FITC-dextran) was added into the inner chamber, and leakage to the outer chamber was detected. The permeability of FITC-dextran was remarkably lower in the transwell in which dUCs were cultured than the cell-free control group and the other control group in which aHDFs were cultured (Fig. 15e). Leakage of FITC-dextran was also reduced for HUCs and finally differentiated HUCs (Fig. 21). This suggests that P4 dUCs produced by the method of the present invention formed an epithelial barrier in vitro.

Example 6

RNA sequence further confirmed characteristics of P2 dUCs as urothelial cells.

Similarity between P2 dUCs and urothelial cells was analyzed by RNA sequencing. Gene expression profiling analysis by hierarchical clustering of full-length genome clarified that dUCs were more similar to HUCs than to aHDFs (Fig. 16A). Fig. 16B shows data of the most variable fifty genes. The results show that both dUCs and HUCs characteristically express keratin (KRT8, 18, 5, 17, 6A, 14), which is rarely expressed in fibroblasts, and genes associated with urothelial cells including RAB27B, which is involved in the transport of uroprakin to the upper cell. In contrast, fibroblast-related genes (FAP, COL16A1, COL1A2, DPT, PTX3, etc.) showed decreased expression in dUCs and HUCs (Fig. 16B). Gene expression analysis further confirmed that dUCs strongly expressed genes related to urothelial cell, not fibroblast (Fig. 16C, Fig. 17A). As for genes related to keratin filament and collagen fiber tissue, HUCs and dUCs showed similar expression patterns (Fig. 17A). What should be noted is that dUCs expressed KLF5 that is one of the important factors related to the development of bladder epithelial cells (Fig. 16C). It was also found that dUCs strongly expressed genes related to the formation of interepithelial cell-cell adhesion tight junction by KEGG pathway analysis (Fig. 17B). These genes also are scarcely expressed in fibroblasts.

Example 7

dUCs may contribute to regeneration of damaged urothelial cell in vivo.

Whether aHDFs into which F, T, L, and K were introduced are converted to dUCs in vivo and contribute to regeneration of urothelial cell was examined. aHDFs into which F, T, L, K and GFP genes had been introduced were converted to GFP-labeled dUCs when they were cultured in CnT-Prime medium for 21 days (Figs. 18a, b). For the in vivo experiments, 4 days after the introduction of F, T, L, K, and G genes, the aHDFs were transplanted into the bladder of mice damaged by interstitial cystitis. One week after the transplantation, GFP-labeled cells were present in the mucosal epithelium of the bladder, and expressed UPK1b, UPK2, E-cadherin, keratin 8/18 (Fig. 18d). In addition, they also expressed KRT20 that is generally expressed in terminally differentiated umbrella cells. In contrast, transplantation of GFP-labeled aHDFs into the bladder did not result in observation of GFP-positive and E-cadherin-positive cells (Fig. 18d). Therefore, aHDFs into which F, L, T, K, and G had been introduced were converted to dUCs in vivo, localized in the epithelium the damaged bladder, and involved in the regeneration of the tissue.

Example 8

Study of presence or absence of tumorigenicity of dUCs

The potential tumorigenicity of dUCs was investigated. In the previous tests, aHDFs into which F, L, T, and K had been introduced did not form tumor even after subcutaneous inoculation to immunodeficient mice (Fig. 19). On the other hand, T24 bladder cancer cells formed tumor.

Example 9

In the same manner as in Example 1, it was confirmed that the urothelial cell-like phenotype was expressed by introducing a specific factor into aHDFs. The results are shown in Fig. 20. It was shown that F, T, L, K or a combination thereof is effective in inducing the expression of UPK1b and UPK2.

[Industrial Applicability]

According to the present invention, urothelial cells can be prepared from somatic cells in a short period of time by direct reprogramming. Since the urothelial cells can be easily derived from the somatic cells of the person who receives transplantation, problems such as immunological rejection and the like do not occur when the obtained urothelial cells are transplanted. In addition, since urothelial cells can be directly induced from somatic cells without going through iPS cells and ES cells, problems caused by pluripotent stem cells such as canceration and the like can be avoided.
This application is based on a patent application No. 2019-159070 filed in Japan (filing date: August 30, 2019), the contents of which are incorporated in full herein.

## Claims

**1.** A method for introducing a urothelial cell, comprising a step of introducing at least one member selected from the group consisting of

FOXA1 (Forkhead box A1) gene or an expression product thereof,
TP63 (tumor protein P63) gene or an expression product thereof,
MYCL (L-Myc) gene or an expression product thereof, and
KLF4 (Kruppel-like factor 4) gene or an expression product thereof
to a mammalian somatic cell as an exogenous factor.

**2.** The method according to claim 1, wherein the exogenous factor comprises FOXA1 gene or an expression product thereof, and TP63 gene or an expression product thereof.

3. The method according to claim 1, wherein the exogenous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

4. The method according to any one of claims 1 to 3, wherein the aforementioned somatic cell is human fibroblast.

5. A urothelial cell derived from a mammalian somatic cell and having at least one member selected from the group consisting of

FOXA1 gene or an expression product thereof,
TP63 gene or an expression product thereof,
MYCL gene or an expression product thereof, and
KLF4 gene or an expression product thereof,
as an exogenous factor.

6. The cell according to claim 5, wherein the exogenous factor comprises FOXA1 gene or an expression product thereof, and TP63 gene or an expression product thereof.

7. The cell according to claim 5, wherein the exogenous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

8. The cell according to any one of claims 5 to 7, wherein the aforementioned somatic cell is human fibroblast.

9. An agent for converting a mammalian somatic cell to a urothelial cell, comprising at least one member selected from the group consisting of

FOXA1 gene or an expression product thereof,
TP63 gene or an expression product thereof,
MYCL gene or an expression product thereof, and
KLF4 gene or an expression product thereof,
as an exogenous factor.

10. The agent according to claim 9, wherein the exogenous factor comprises FOXA1 gene or an expression product thereof, and TP63 gene or an expression product thereof.

11. The agent according to claim 9, wherein the exogenous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

12. The agent according to any one of claims 9 to 11, wherein the aforementioned somatic cell is human fibroblast.

13. A vector for converting a mammalian somatic cell to a urothelial cell, comprising at least one member selected from the group consisting of

FOXA1 gene or an expression product thereof,
TP63 gene or an expression product thereof,
MYCL gene or an expression product thereof, and
KLF4 gene or an expression product thereof,
as an exogenous factor.

14. The vector according to claim 13, wherein the exogenous factor comprises FOXA1 gene or an expression product thereof, and TP63 gene or an expression product thereof.

15. The vector according to claim 13, wherein the exogenous factor is selected from the following;

(i) KLF4 gene or an expression product thereof
(ii) TP63 gene or an expression product thereof, and KLF4 gene or an expression product thereof
(iii) MYCL gene or an expression product thereof, and KLF4 gene or an expression product thereof
(iv) FOXA1 gene or an expression product thereof, MYCL gene or an expression product thereof, and KLF4 gene or an expression product thereof
(v) TP63 gene or an expression product thereof, MYCL gene or an expression product thereof, and KLF4 gene or an expression product thereof
(vi) FOXA1 gene or an expression product thereof, TP63 gene or an expression product thereof, and KLF4 gene or an expression product thereof
(vii) FOXA1 gene or an expression product thereof, TP63 gene or an expression product thereof, MYCL gene or an expression product thereof, and KLF4 gene or an expression product thereof.

16. The vector according to any one of claims 13 to 15, wherein the aforementioned somatic cell is human fibroblast.

17. A therapeutic agent for a urologic disease, comprising a cell obtained by the method according to any one of claims 1 to 4, a urothelial cell according to any one of claims 5 to 8, an agent according to any one of claims 9 to 12, or a vector according to any one of claims 13 to 16.

18. The therapeutic agent according to claim 17, wherein the urologic disease is a urinary disease.

19. The therapeutic agent according to claim 18, wherein the urinary disease is at least one member selected from the group consisting of bladder cancer, overactive bladder, congenital bladder anomalies, bladder injury, interstitial cystitis, neurogenic bladder and contracted bladder.

20. A medium for urothelial cell induction, comprising 3-isobutyl 1-methylxanthine (IBMX) and epidermal growth factor (EGF) .

FIG. 1

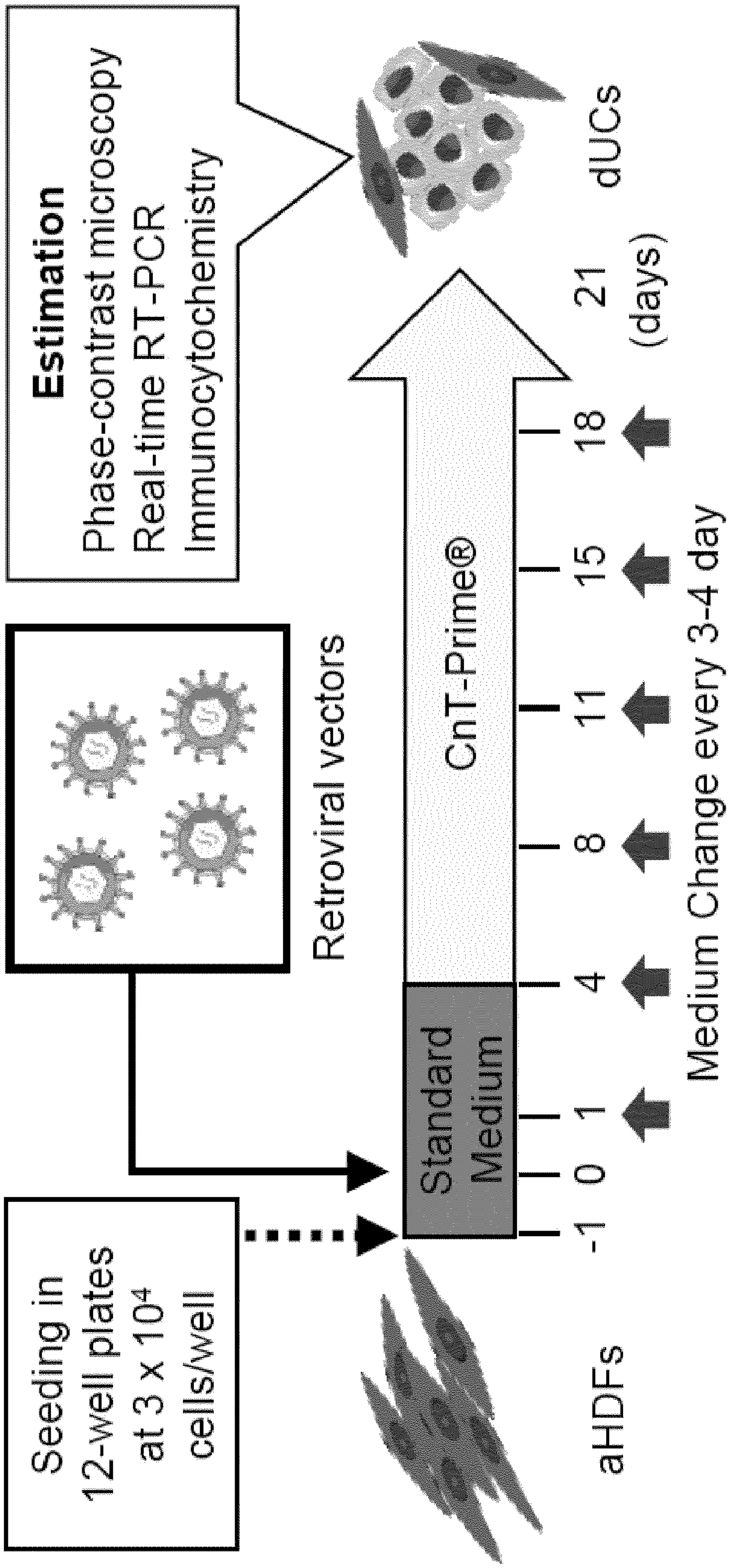
Seeding in 12-well plates at 3 x 10$^4$ cells/well
aHDFs
Retroviral vectors
Standard Medium
CnT-Prime®
-1
0
1
4
8
11
15
18
21
(days)
Medium Change every 3-4 day
Estimation
Phase-contrast microscopy
Real-time RT-PCR
Immunocytochemistry
dUCs

FIG. 2

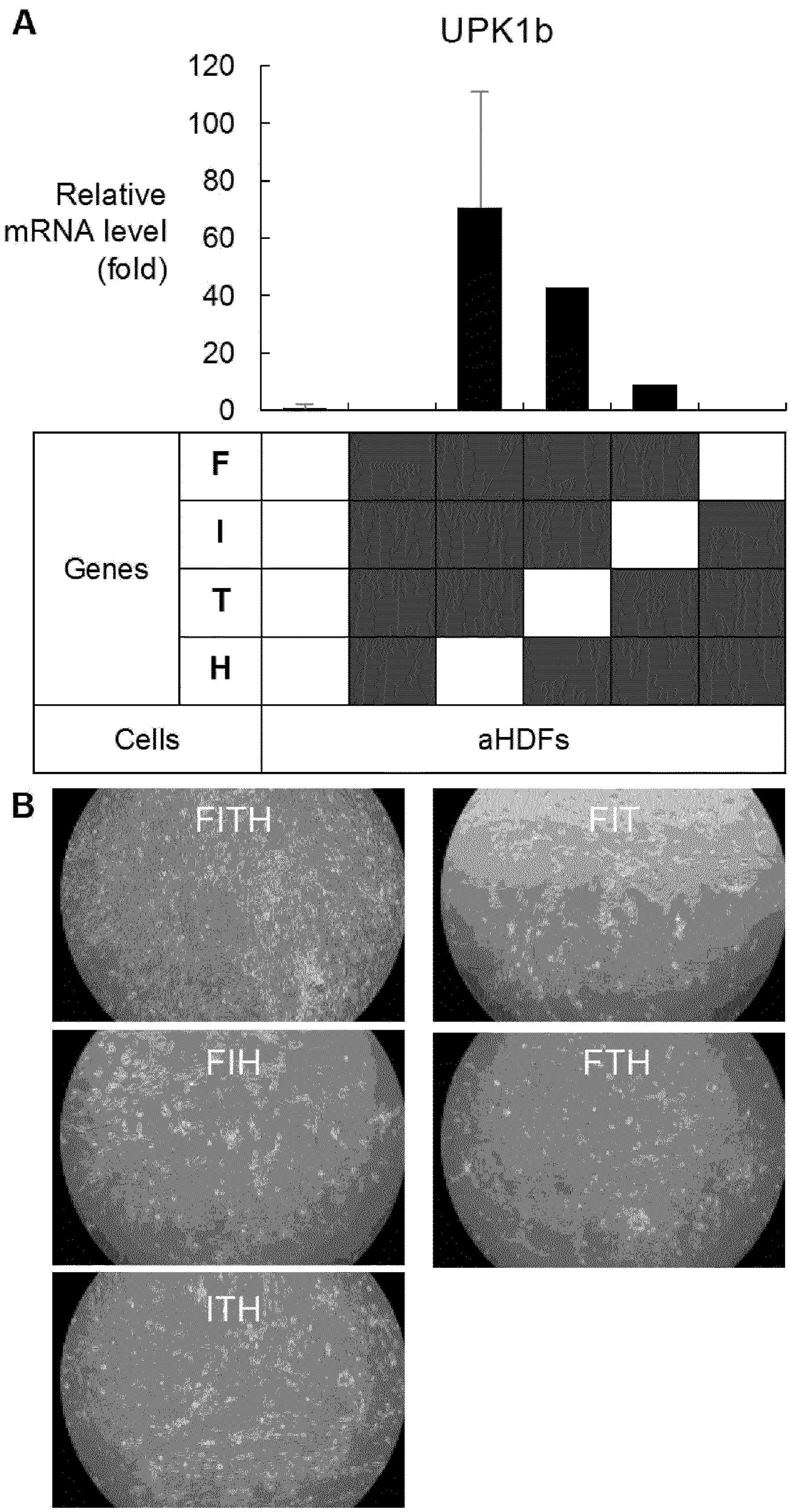
A
UPK1b
Relative
mRNA level
(fold)
120
100
80
60
40
20
0
Genes
F
I
T
H
Cells
aHDFs
B
FITH
FIT
FIH
FTH
ITH

FIG. 3

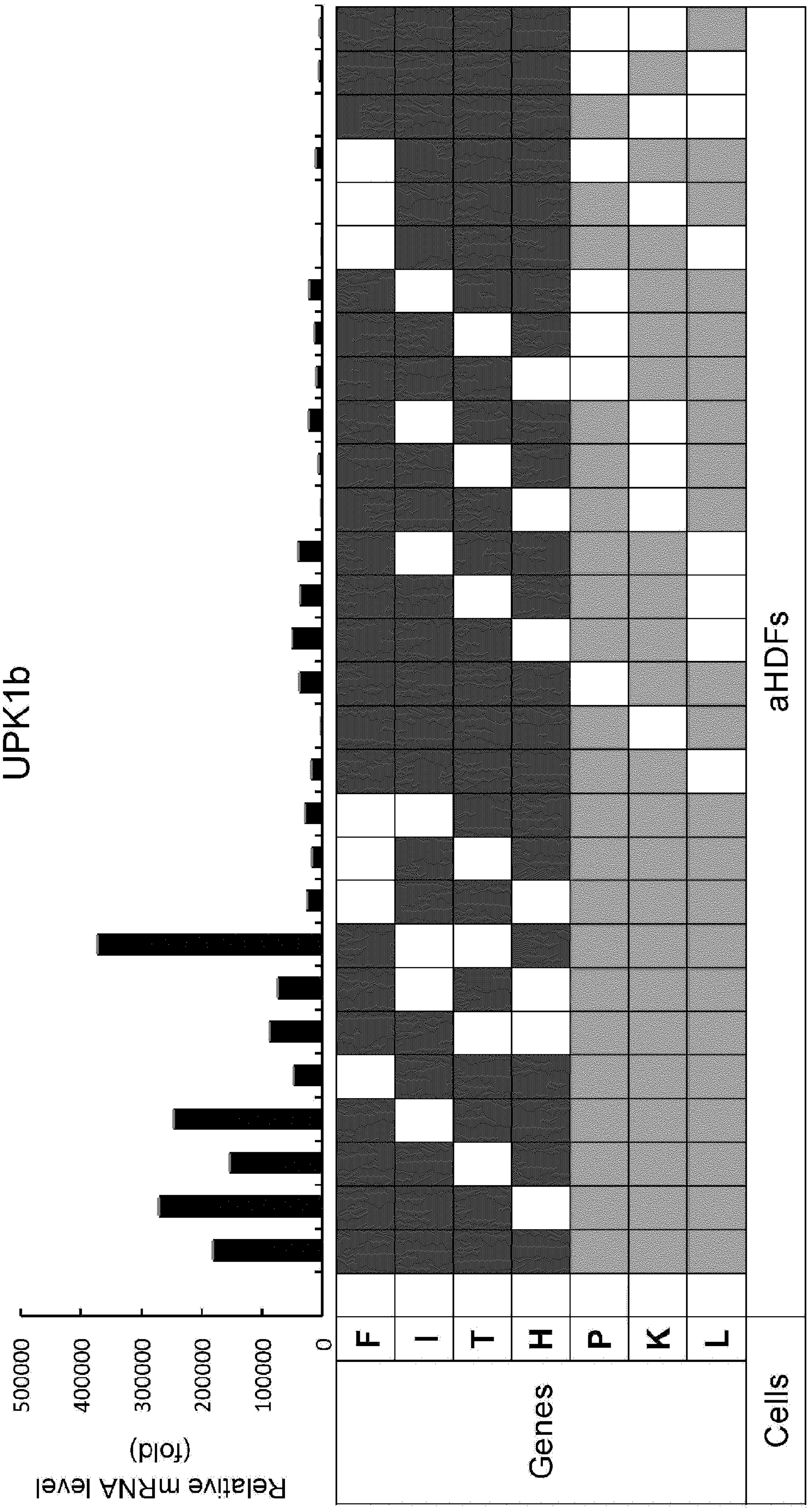
UPK1b
Relative mRNA level (fold)
500000
400000
300000
200000
100000
0
Genes
F
I
T
H
P
K
L
Cells
aHDFs

FIG. 4

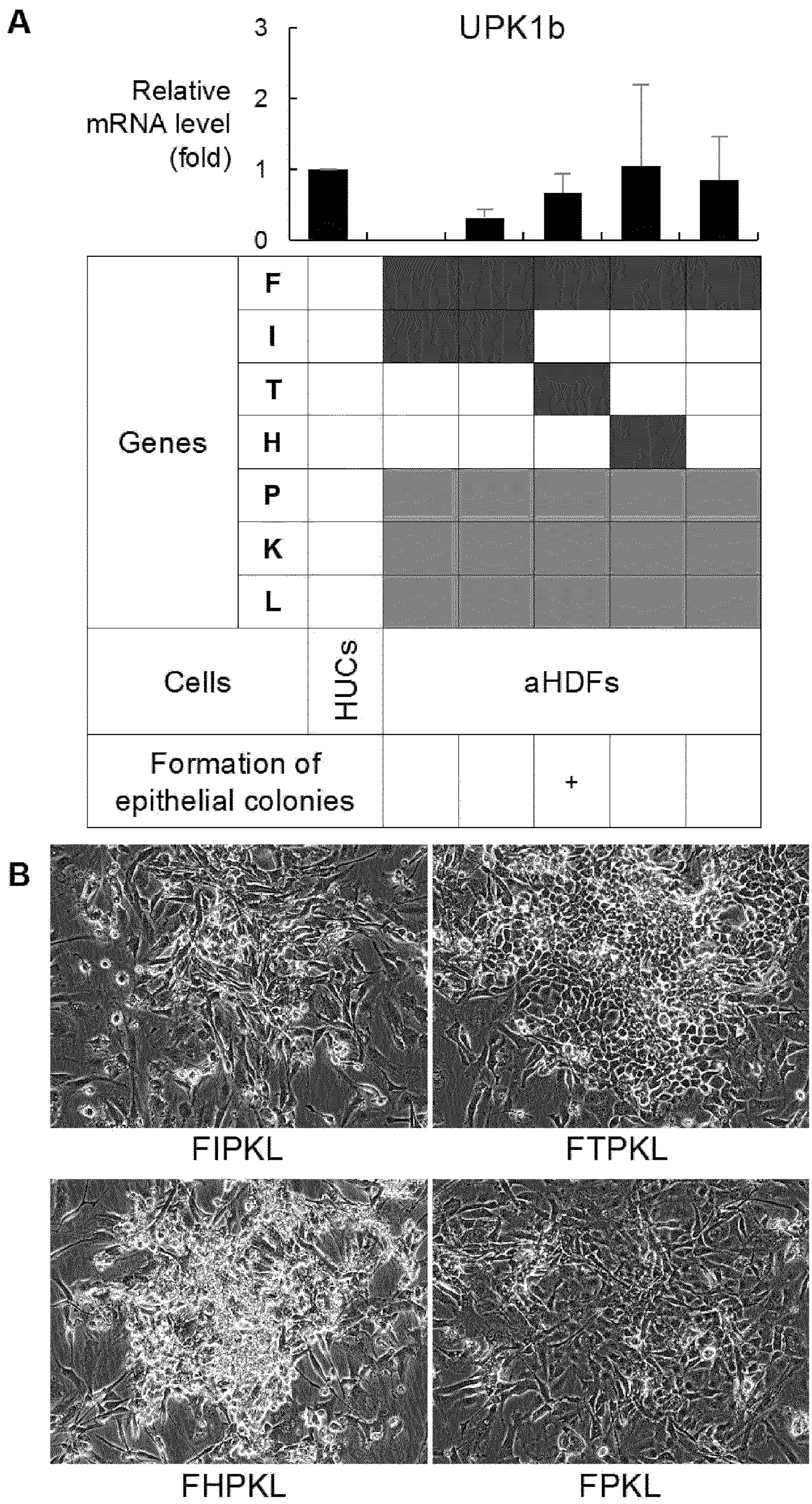
A
UPK1b
Relative mRNA level (fold)
3
2
1
0
Genes
F
I
T
H
P
K
L
Cells
HUCs
aHDFs
Formation of epithelial colonies
+
B
FIPKL
FTPKL
FHPKL
FPKL

FIG. 5

A

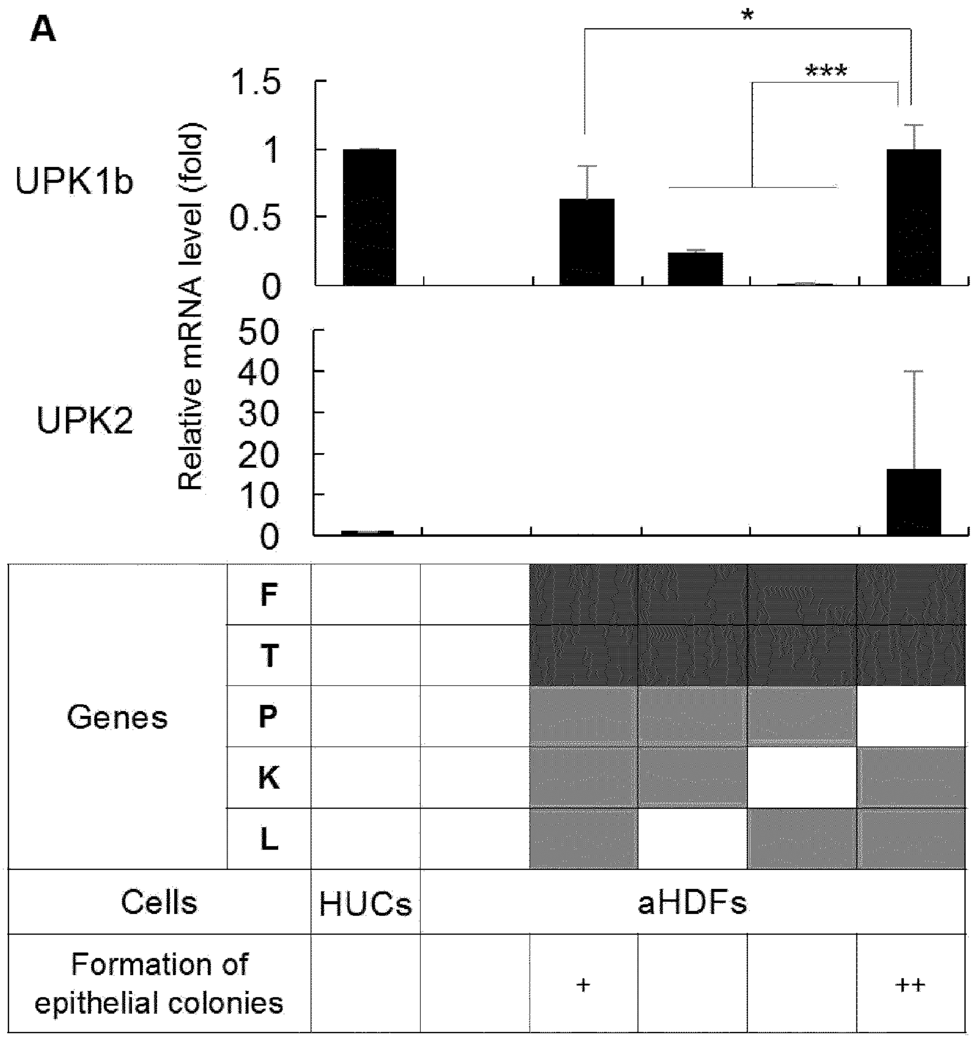
*
***
1.5
1
0.5
0
UPK1b
Relative mRNA level (fold)
50
40
30
20
10
0
UPK2
Genes
F
T
P
K
L
Cells
HUCs
aHDFs
Formation of epithelial colonies
+
++

B

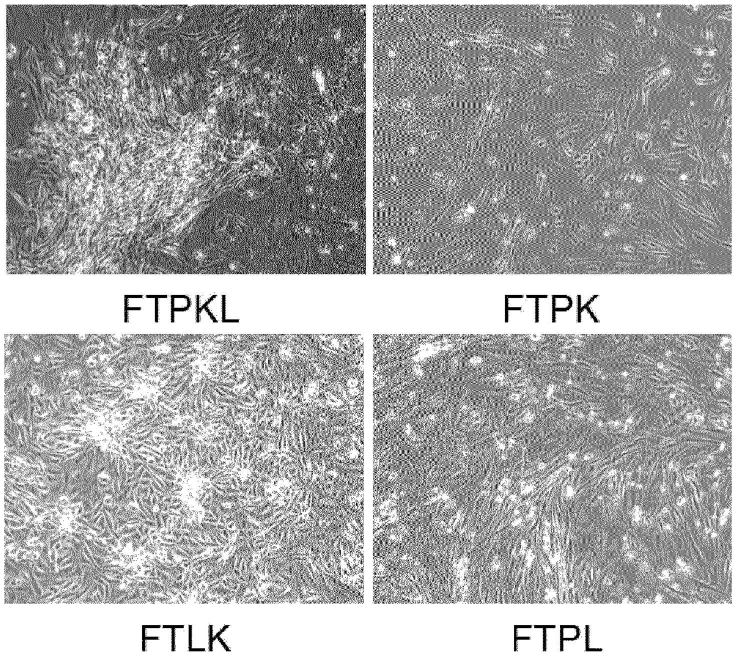
FTPKL
FTPK
FTLK
FTPL

FIG. 6

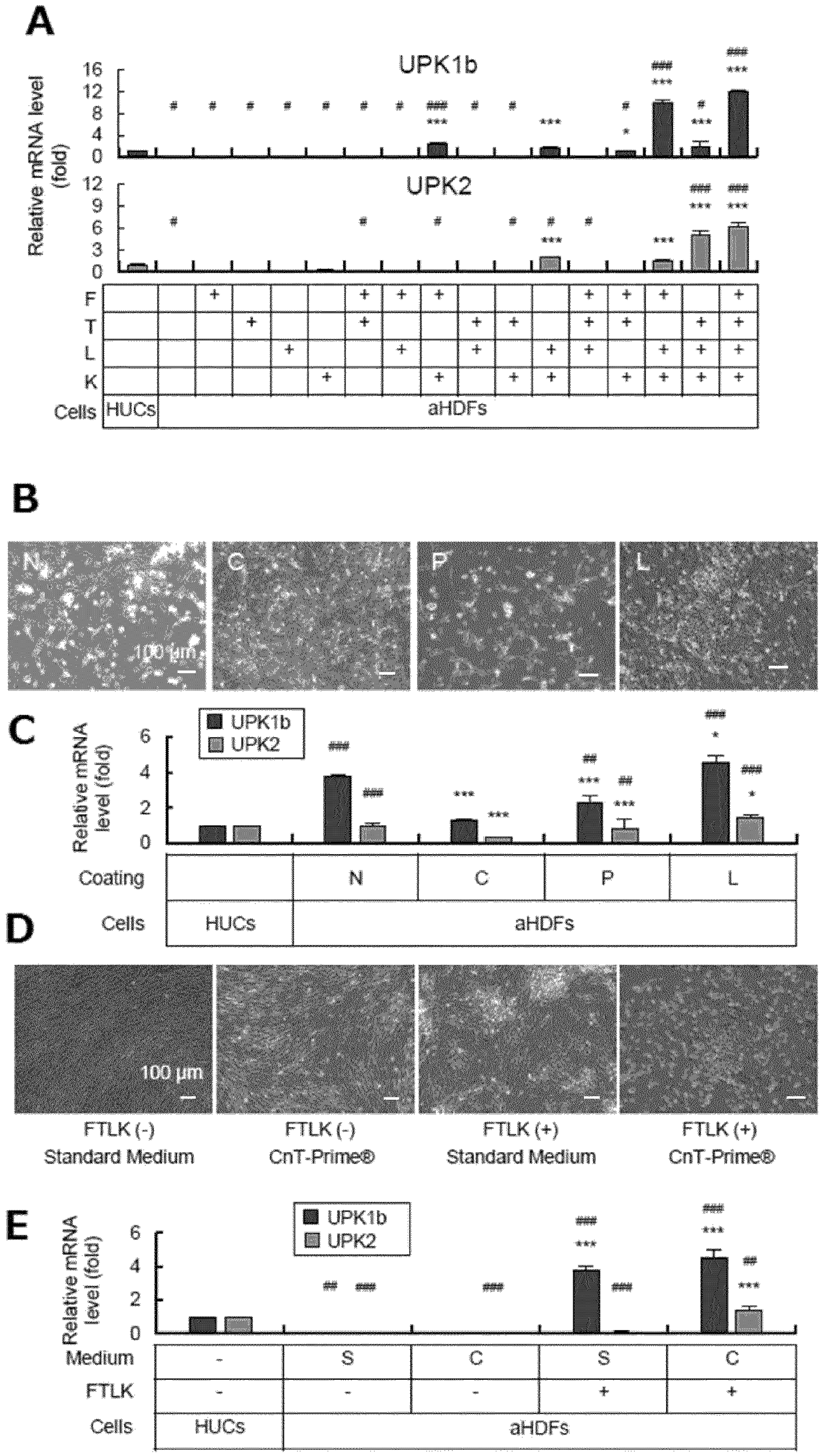
A
UPK1b
UPK2
Relative mRNA level (fold)
F
T
L
K
Cells
HUCs
aHDFs
B
N
C
P
L
100 μm
C
UPK1b
UPK2
Relative mRNA level (fold)
Coating
N
C
P
L
Cells
HUCs
aHDFs
D
100 μm
FTLK (-) Standard Medium
FTLK (-) CnT-Prime®
FTLK (+) Standard Medium
FTLK (+) CnT-Prime®
E
UPK1b
UPK2
Relative mRNA level (fold)
Medium
S
C
FTLK
Cells
HUCs
aHDFs

FIG. 7

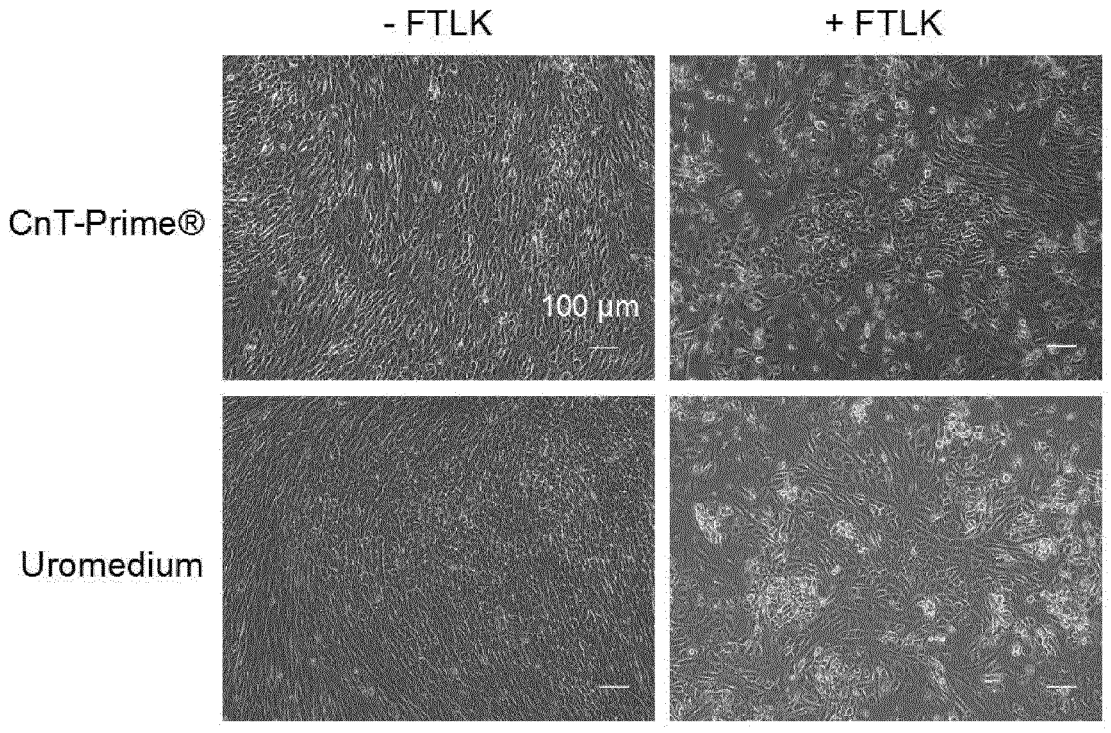

FIG. 8

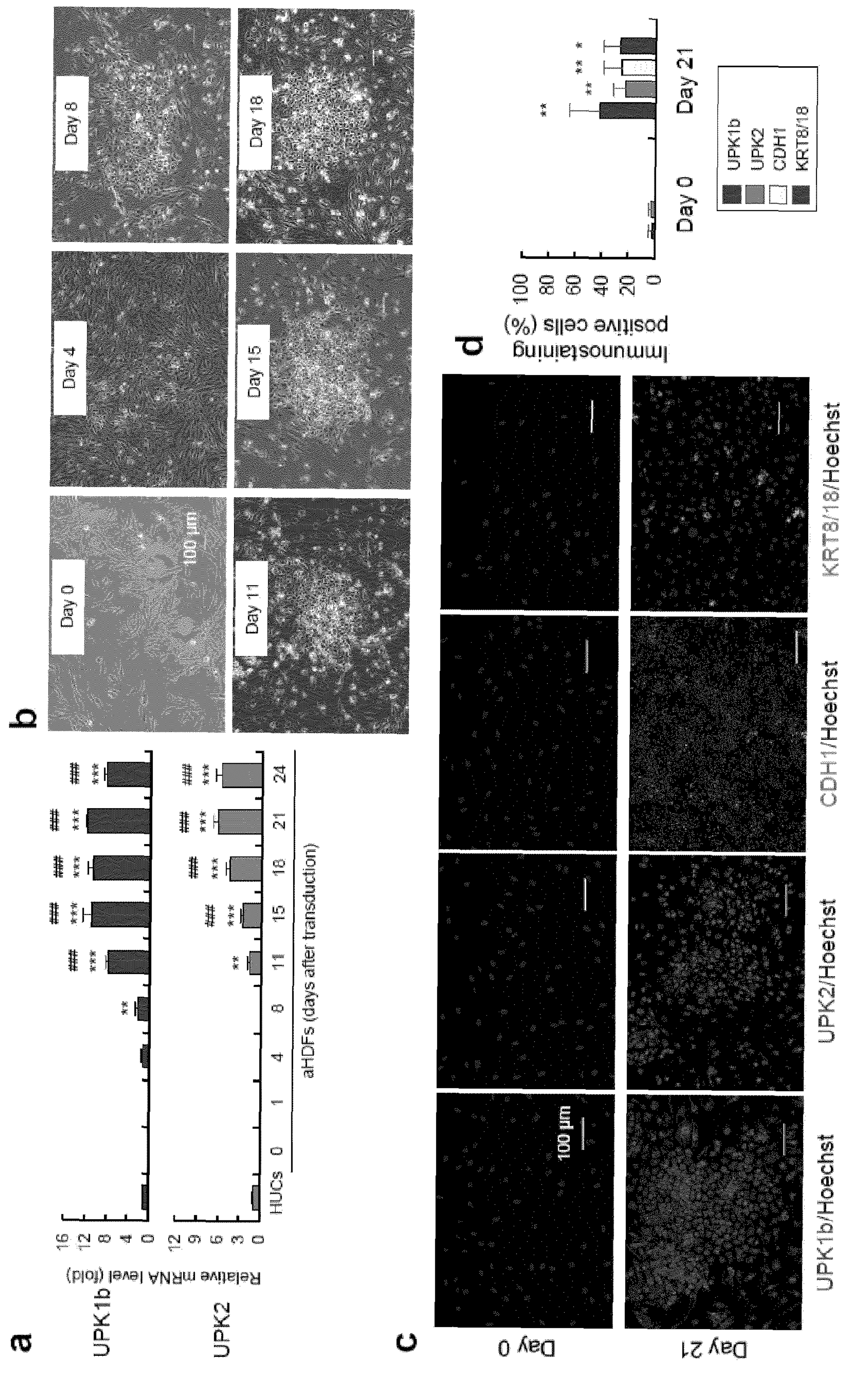
a
UPK1b
UPK2
Relative mRNA level (fold)
16
12
8
4
0
12
9
6
3
0
HUCs
0
1
4
8
11
15
18
21
24
aHDFs (days after transduction)
**
***
###
b
Day 0
Day 4
Day 8
Day 11
Day 15
Day 18
100 μm
c
Day 0
Day 21
UPK1b/Hoechst
UPK2/Hoechst
CDH1/Hoechst
KRT8/18/Hoechst
100 μm
d
Immunostaining positive cells (%)
100
80
60
40
20
0
Day 0
Day 21
UPK1b
UPK2
CDH1
KRT8/18

FIG. 9

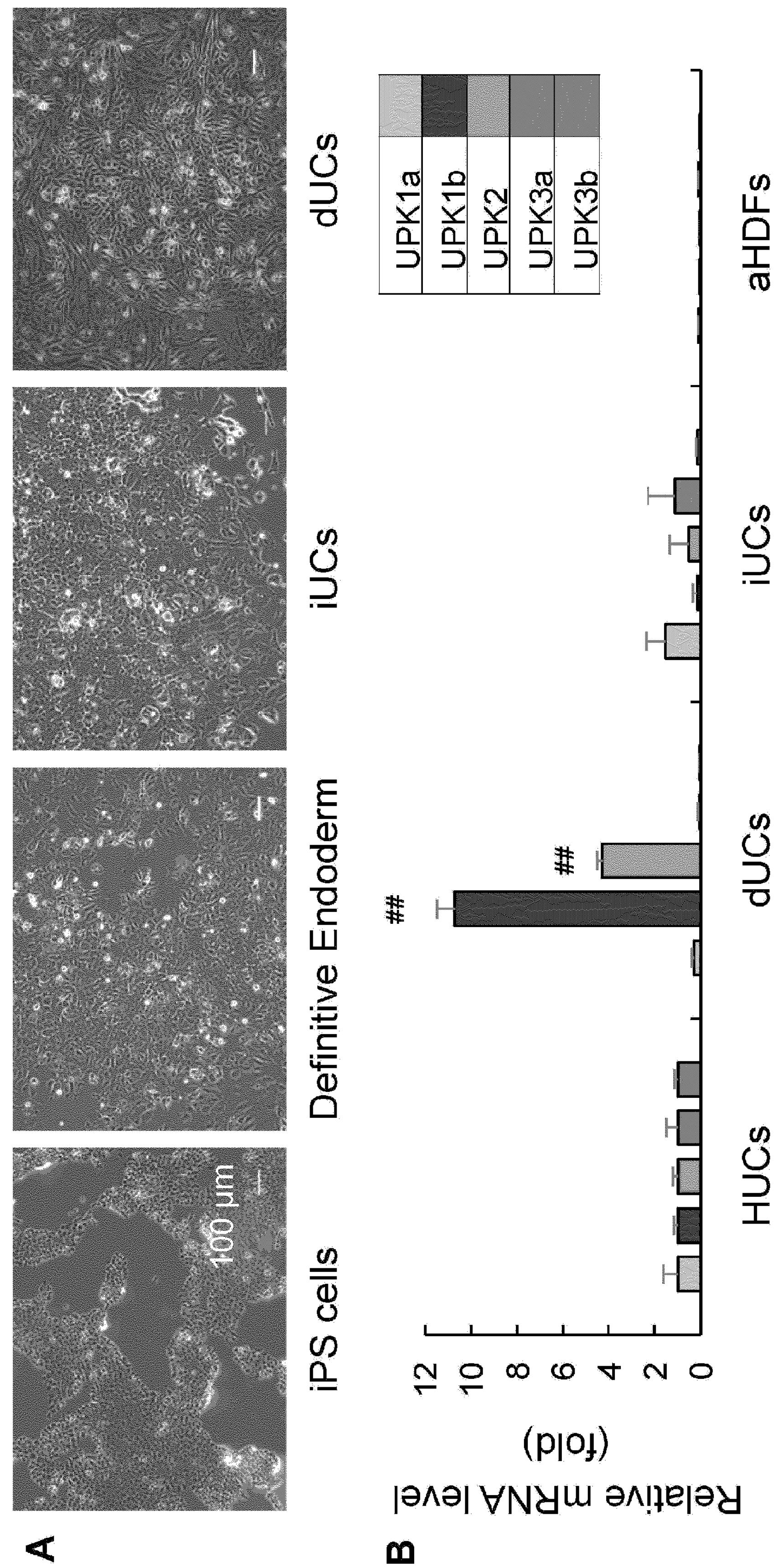
A
iPS cells
Definitive Endoderm
iUCs
dUCs
100 μm
B
Relative mRNA level (fold)
0
2
4
6
8
10
12
##
##
HUCs
dUCs
iUCs
aHDFs
UPK1a
UPK1b
UPK2
UPK3a
UPK3b

FIG. 10

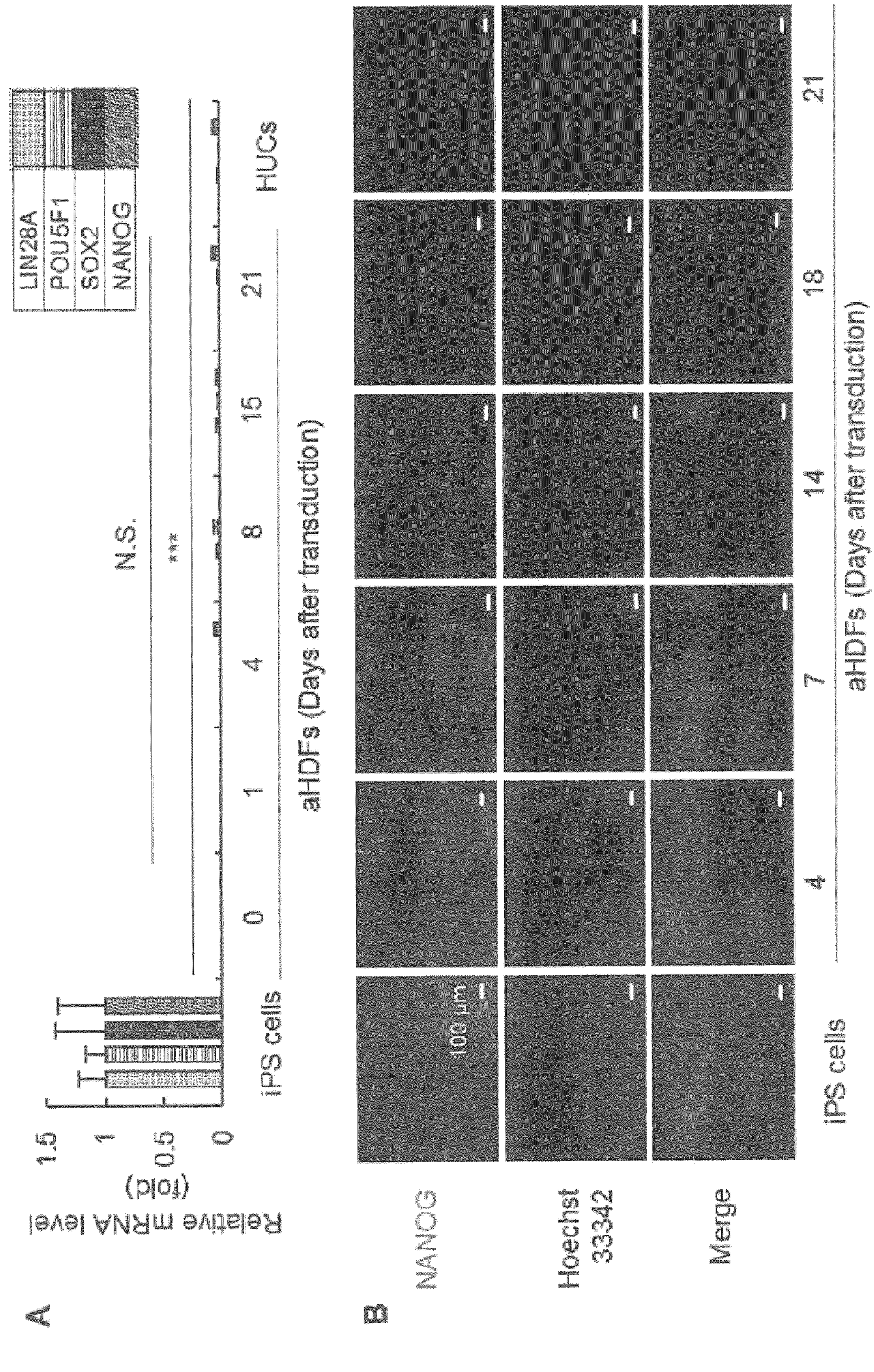
A
Relative mRNA level (fold)
1.5
1
0.5
0
LIN28A
POU5F1
SOX2
NANOG
N.S.
***
iPS cells
0
1
4
8
15
21
aHDFs (Days after transduction)
HUCs
B
NANOG
Hoechst 33342
Merge
100 μm
iPS cells
4
7
14
18
21
aHDFs (Days after transduction)

FIG. 11

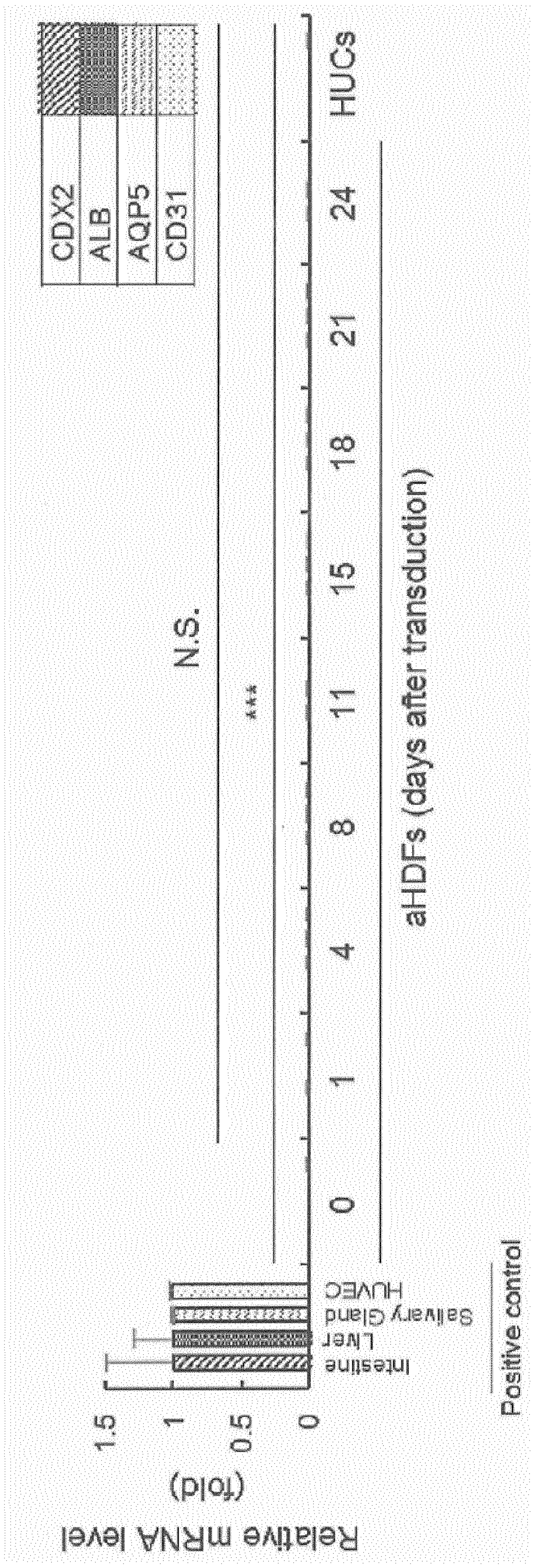
Relative mRNA level
(fold)
1.5
1
0.5
0
Intestine
Liver
Salivary Gland
HUVEC
Positive control
0
1
4
8
11
15
18
21
24
HUCs
aHDFs (days after transduction)
N.S.
***
CDX2
ALB
AQP5
CD31

FIG. 12

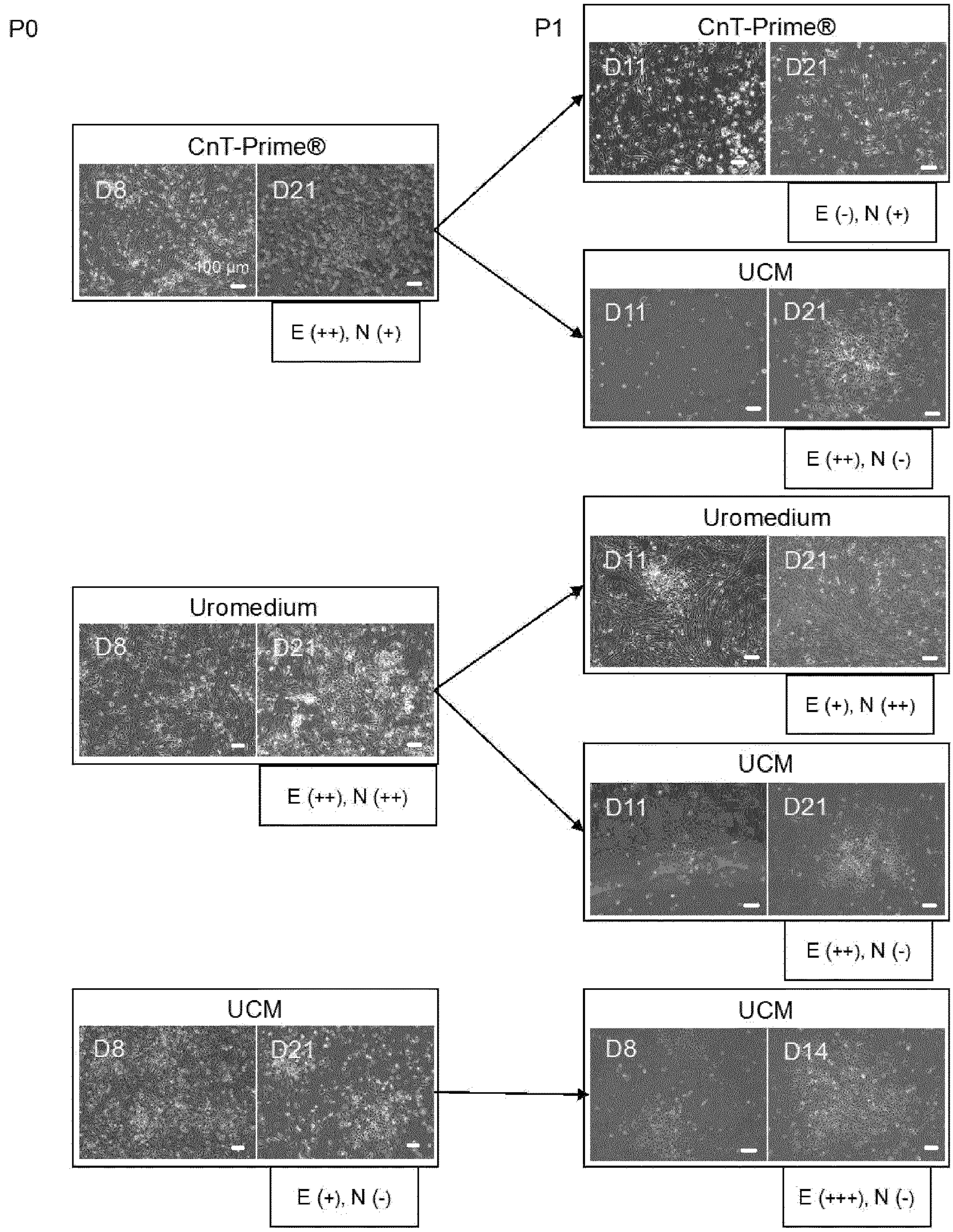
P0
P1
CnT-Prime®
D8
D21
100 µm
E (++), N (+)
CnT-Prime®
D11
D21
E (-), N (+)
UCM
D11
D21
E (++), N (-)
Uromedium
D8
D21
E (++), N (++)
Uromedium
D11
D21
E (+), N (++)
UCM
D11
D21
E (++), N (-)
UCM
D8
D21
E (+), N (-)
UCM
D8
D14
E (+++), N (-)

FIG. 13

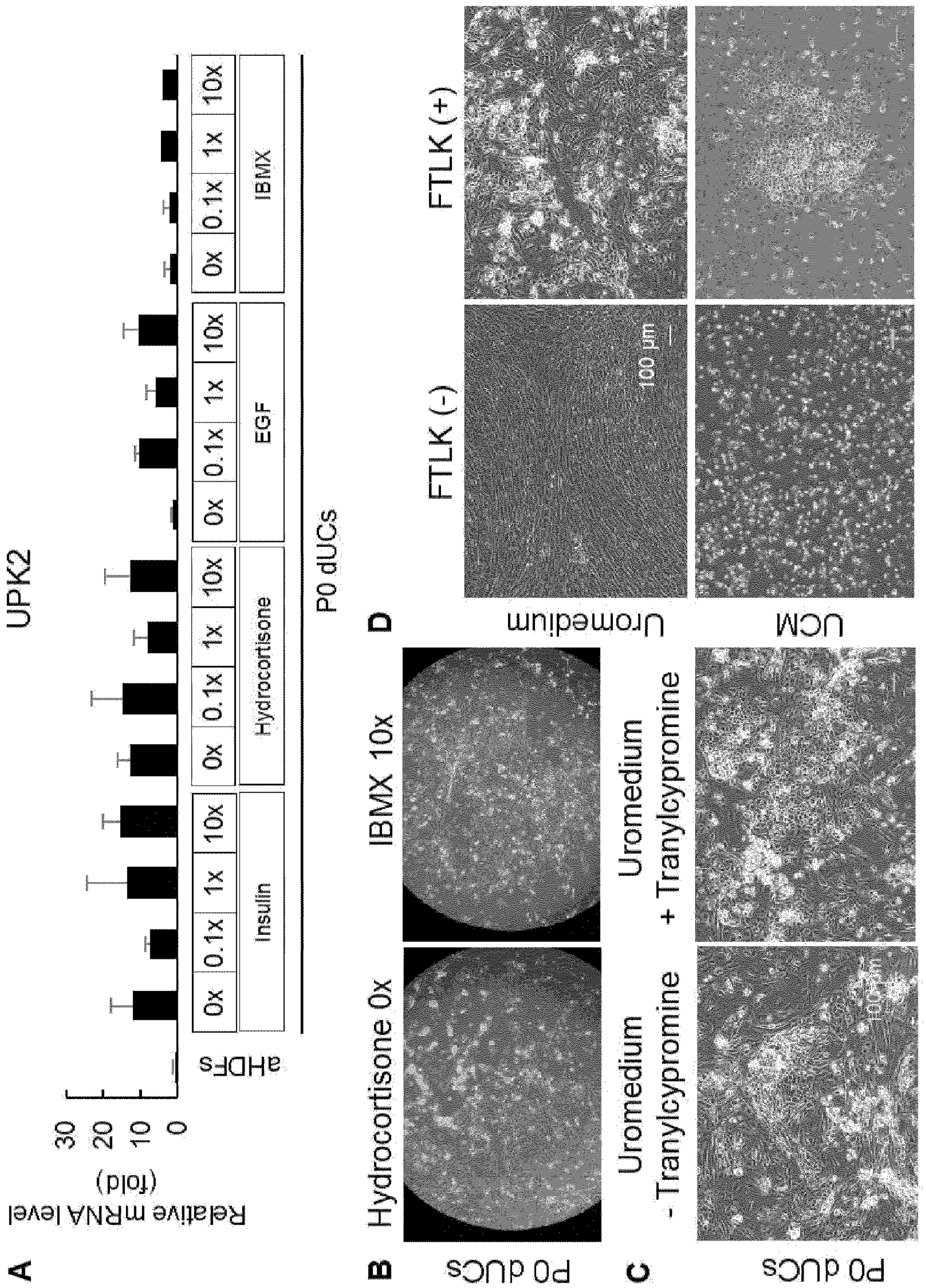
A
UPK2
Relative mRNA level (fold)
30
20
10
0
aHDFs
0x
0.1x
1x
10x
Insulin
Hydrocortisone
EGF
IBMX
P0 dUCs
B
P0 dUCs
Hydrocortisone 0x
IBMX 10x
C
P0 dUCs
Uromedium - Tranylcypromine
Uromedium + Tranylcypromine
100 μm
D
Uromedium
UCM
FTLK (-)
FTLK (+)
100 μm

FIG. 14

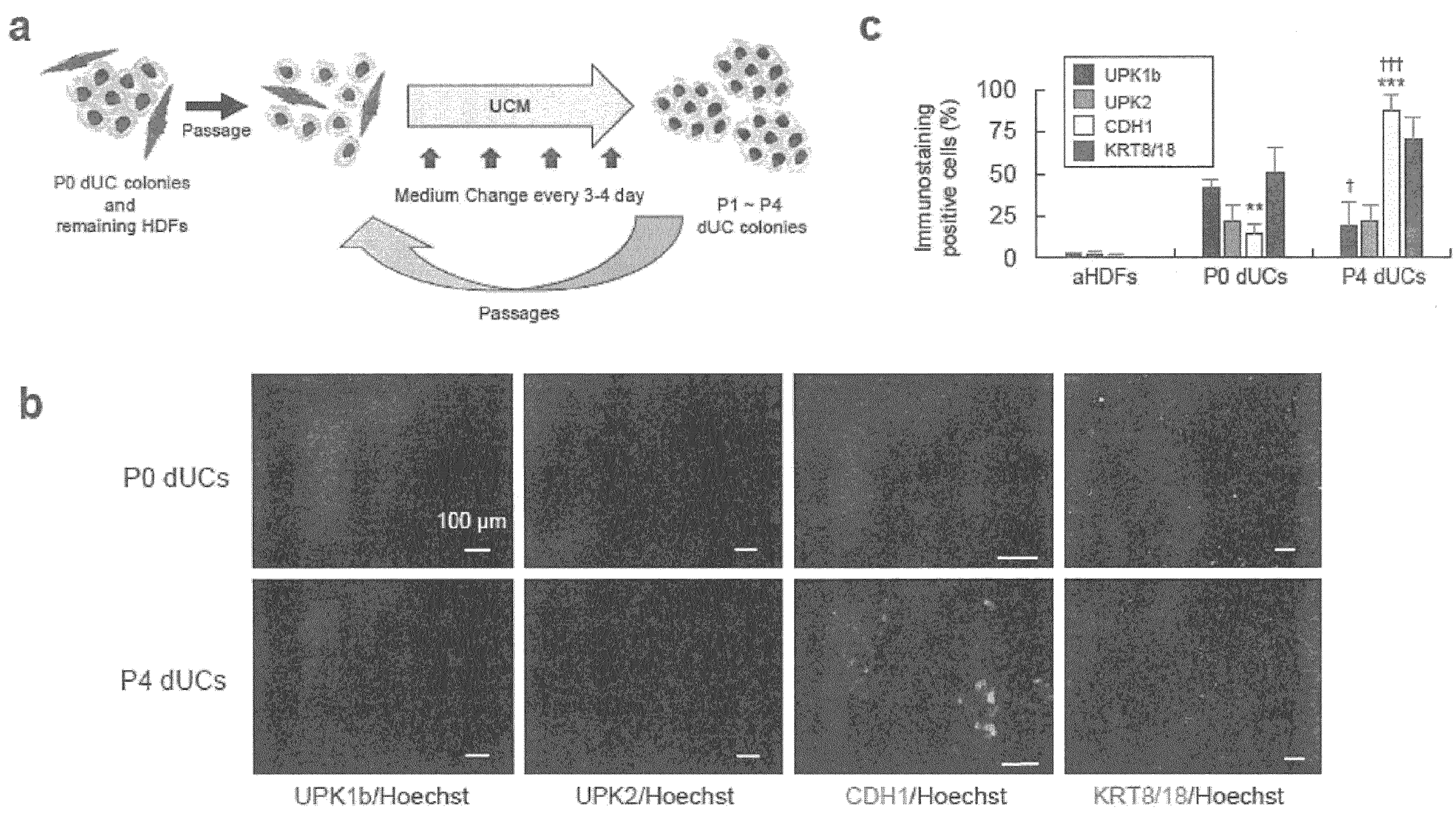
a
P0 dUC colonies
and
remaining HDFs
Passage
UCM
Medium Change every 3-4 day
P1 ~ P4
dUC colonies
Passages
c
Immunostaining
positive cells (%)
100
75
50
25
0
UPK1b
UPK2
CDH1
KRT8/18
aHDFs
P0 dUCs
P4 dUCs
**
†
†††
***
b
P0 dUCs
P4 dUCs
100 μm
UPK1b/Hoechst
UPK2/Hoechst
CDH1/Hoechst
KRT8/18/Hoechst

FIG. 15

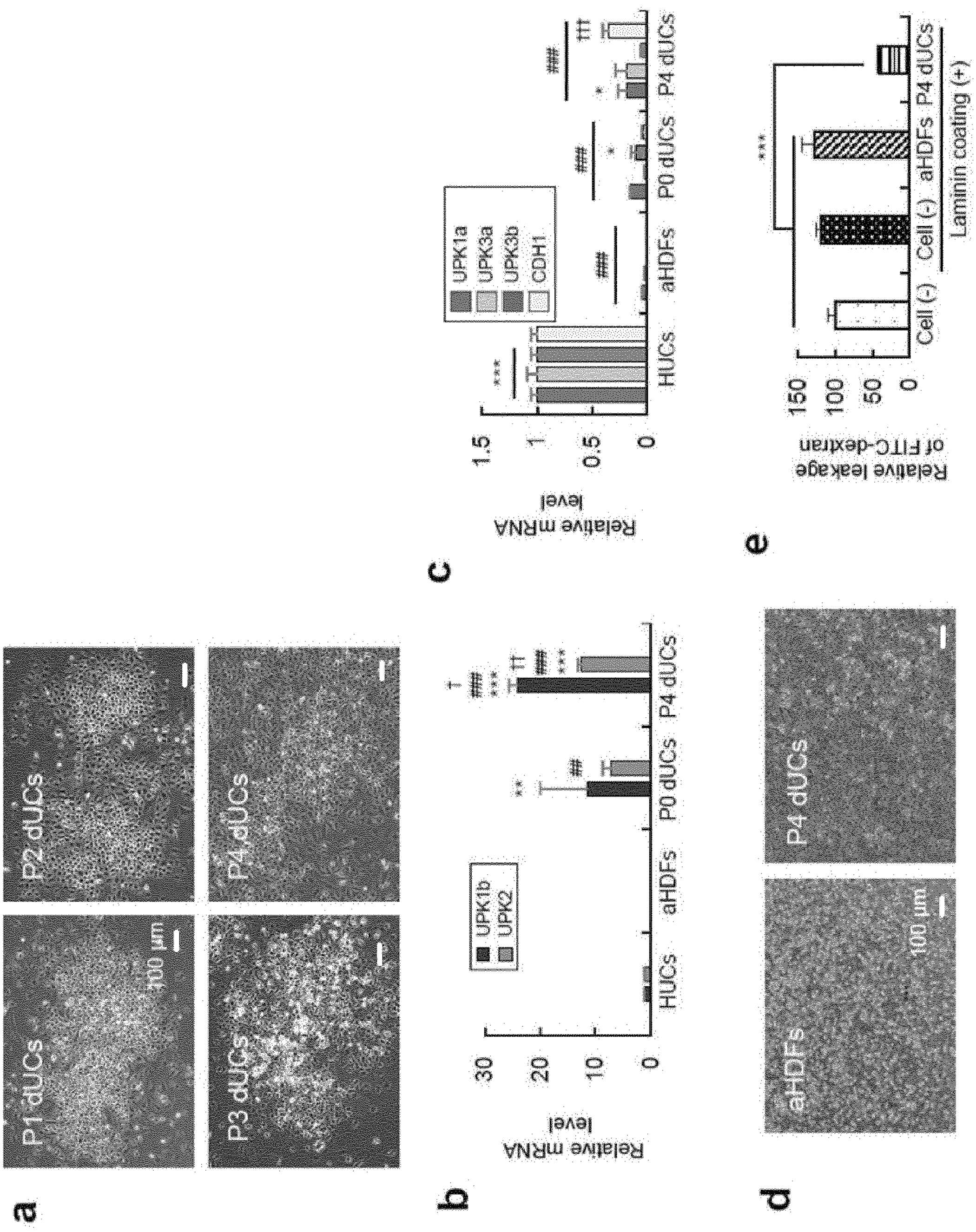
a
P1 dUCs
100 μm
P2 dUCs
P3 dUCs
P4 dUCs
b
Relative mRNA level
UPK1b
UPK2
HUCs
aHDFs
P0 dUCs
P4 dUCs
c
Relative mRNA level
UPK1a
UPK3a
UPK3b
CDH1
HUCs
aHDFs
P0 dUCs
P4 dUCs
d
aHDFs
100 μm
P4 dUCs
e
Relative leakage of FITC-dextran
Cell (-)
Cell (-)
aHDFs
P4 dUCs
Laminin coating (+)

FIG. 16

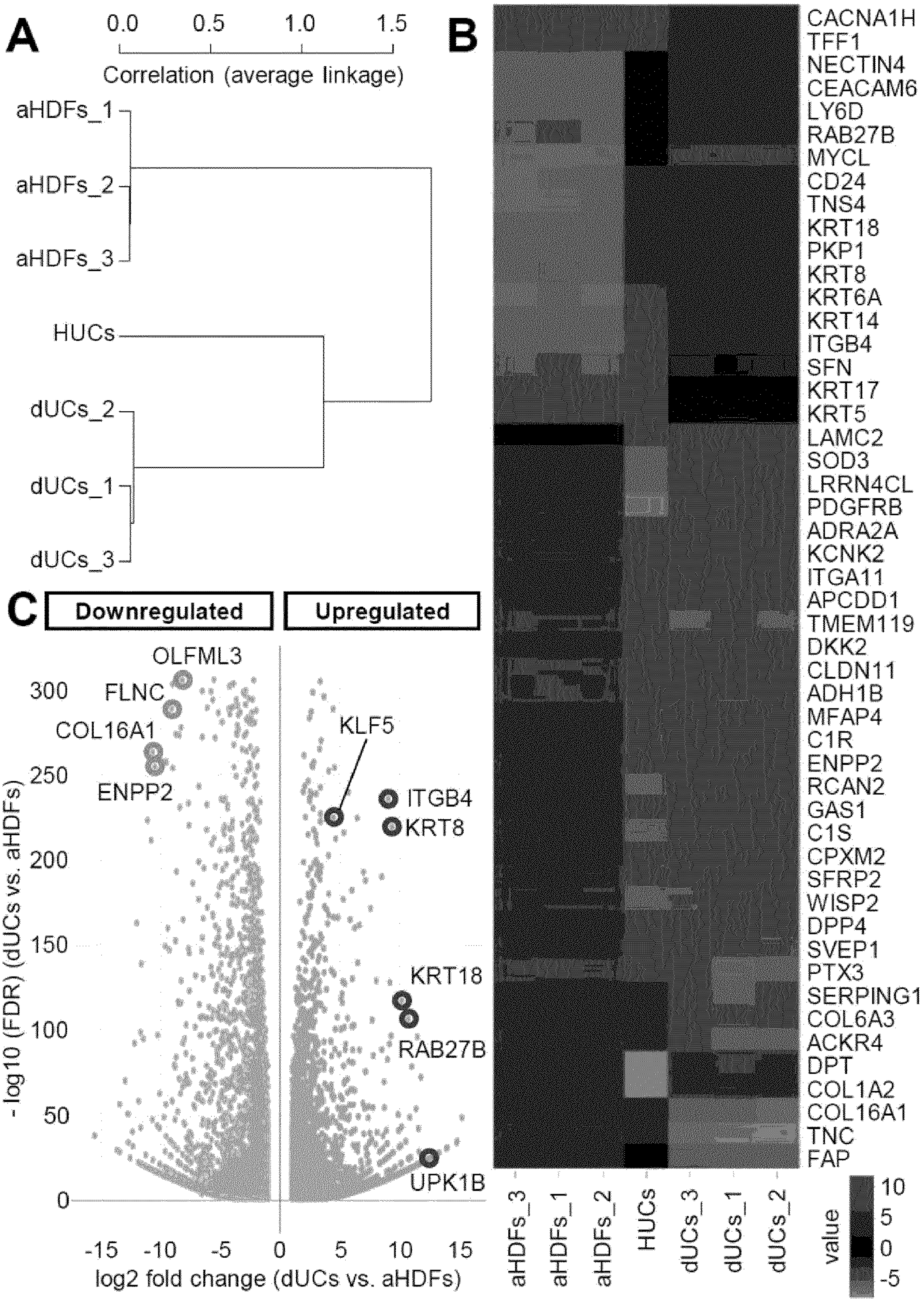
A
0.0
0.5
1.0
1.5
Correlation (average linkage)
aHDFs_1
aHDFs_2
aHDFs_3
HUCs
dUCs_2
dUCs_1
dUCs_3
B
CACNA1H
TFF1
NECTIN4
CEACAM6
LY6D
RAB27B
MYCL
CD24
TNS4
KRT18
PKP1
KRT8
KRT6A
KRT14
ITGB4
SFN
KRT17
KRT5
LAMC2
SOD3
LRRN4CL
PDGFRB
ADRA2A
KCNK2
ITGA11
APCDD1
TMEM119
DKK2
CLDN11
ADH1B
MFAP4
C1R
ENPP2
RCAN2
GAS1
C1S
CPXM2
SFRP2
WISP2
DPP4
SVEP1
PTX3
SERPING1
COL6A3
ACKR4
DPT
COL1A2
COL16A1
TNC
FAP
aHDFs_3
aHDFs_1
aHDFs_2
HUCs
dUCs_3
dUCs_1
dUCs_2
value
10
5
0
-5
C
Downregulated
Upregulated
OLFML3
FLNC
COL16A1
ENPP2
KLF5
ITGB4
KRT8
KRT18
RAB27B
UPK1B
300
250
200
150
100
50
0
- log10 (FDR) (dUCs vs. aHDFs)
-15
-10
-5
0
5
10
15
log2 fold change (dUCs vs. aHDFs)

FIG. 17A

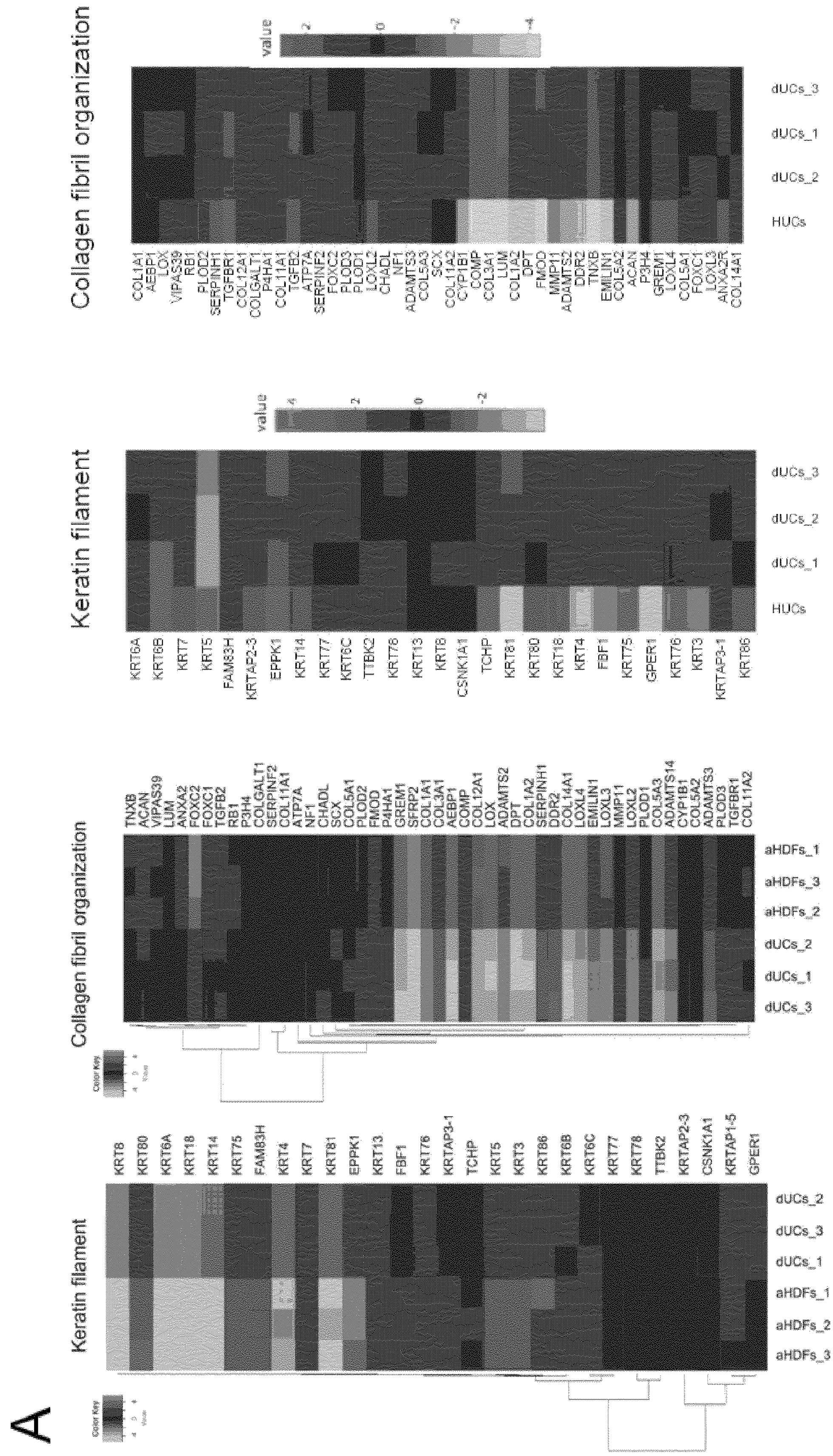
A
Keratin filament
Color Key
Value
KRT8 KRT80 KRT6A KRT18 KRT14 KRT75 FAM83H KRT4 KRT7 KRT81 EPPK1 KRT13 FBF1 KRT76 KRTAP3-1 TCHP KRT5 KRT3 KRT86 KRT6B KRT6C KRT77 KRT78 TTBK2 KRTAP2-3 CSNK1A1 KRTAP1-5 GPER1
dUCs_2 dUCs_3 dUCs_1 aHDFs_1 aHDFs_2 aHDFs_3
Collagen fibril organization
TNXB ACAN VIPAS39 LUM ANXA2 FOXC2 FOXC1 TGFB2 RB1 P3H4 COLGALT1 SERPINF2 COL11A1 ATP7A NF1 CHADL SCX COL5A1 PLOD2 FMOD P4HA1 GREM1 SFRP2 COL1A1 COL3A1 AEBP1 COMP COL12A1 LOX ADAMTS2 DPT COL1A2 SERPINH1 DDR2 COL14A1 LOXL4 EMILIN1 LOXL3 MMP11 LOXL2 PLOD1 COL5A3 ADAMTS14 CYP1B1 COL5A2 ADAMTS3 PLOD3 TGFBR1 COL11A2
aHDFs_1 aHDFs_3 aHDFs_2 dUCs_2 dUCs_1 dUCs_3
Keratin filament
value
4 2 0 -2
KRT6A KRT6B KRT7 KRT5 FAM83H KRTAP2-3 EPPK1 KRT14 KRT77 KRT6C TTBK2 KRT78 KRT13 KRT8 CSNK1A1 TCHP KRT81 KRT80 KRT18 KRT4 FBF1 KRT75 GPER1 KRT76 KRT3 KRTAP3-1 KRT86
dUCs_3 dUCs_2 dUCs_1 HUCs
Collagen fibril organization
value
2 0 -2 -4
COL1A1 AEBP1 LOX VIPAS39 RB1 PLOD2 SERPINH1 TGFBR1 COL12A1 COLGALT1 P4HA1 COL11A1 TGFB2 ATP7A SERPINF2 FOXC2 PLOD3 PLOD1 LOXL2 CHADL NF1 ADAMTS3 COL5A3 SCX COL11A2 CYP1B1 COMP COL3A1 LUM COL1A2 DPT FMOD MMP11 ADAMTS2 DDR2 TNXB EMILIN1 COL5A2 ACAN P3H4 GREM1 LOXL4 COL5A1 FOXC1 LOXL3 ANXA2R COL14A1
dUCs_3 dUCs_1 dUCs_2 HUCs

FIG. 17B

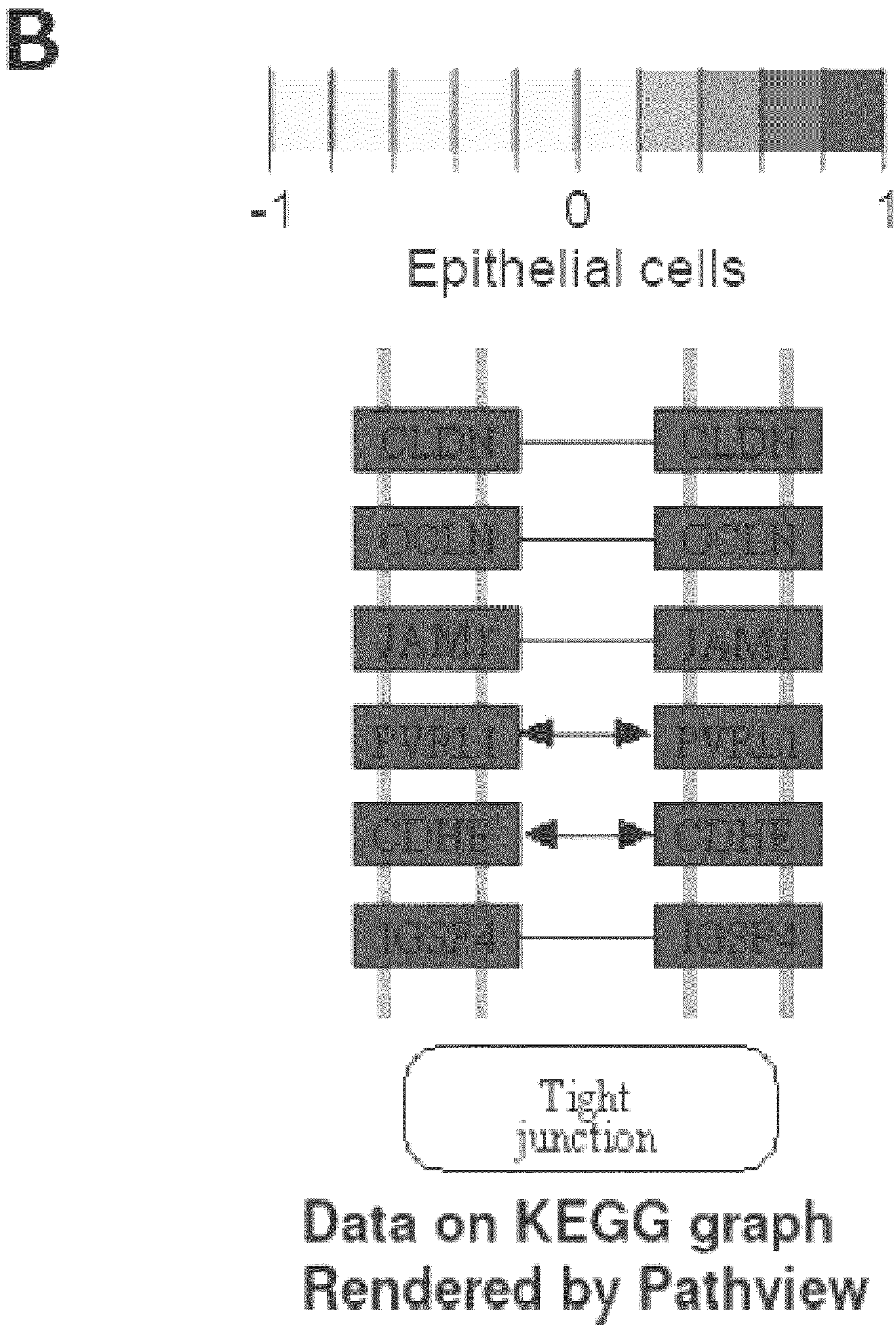
B
-1
0
1
Epithelial cells
CLDN
CLDN
OCLN
OCLN
JAM1
JAM1
PVRL1
PVRL1
CDHE
CDHE
IGSF4
IGSF4
Tight junction
Data on KEGG graph
Rendered by Pathview

FIG. 18A

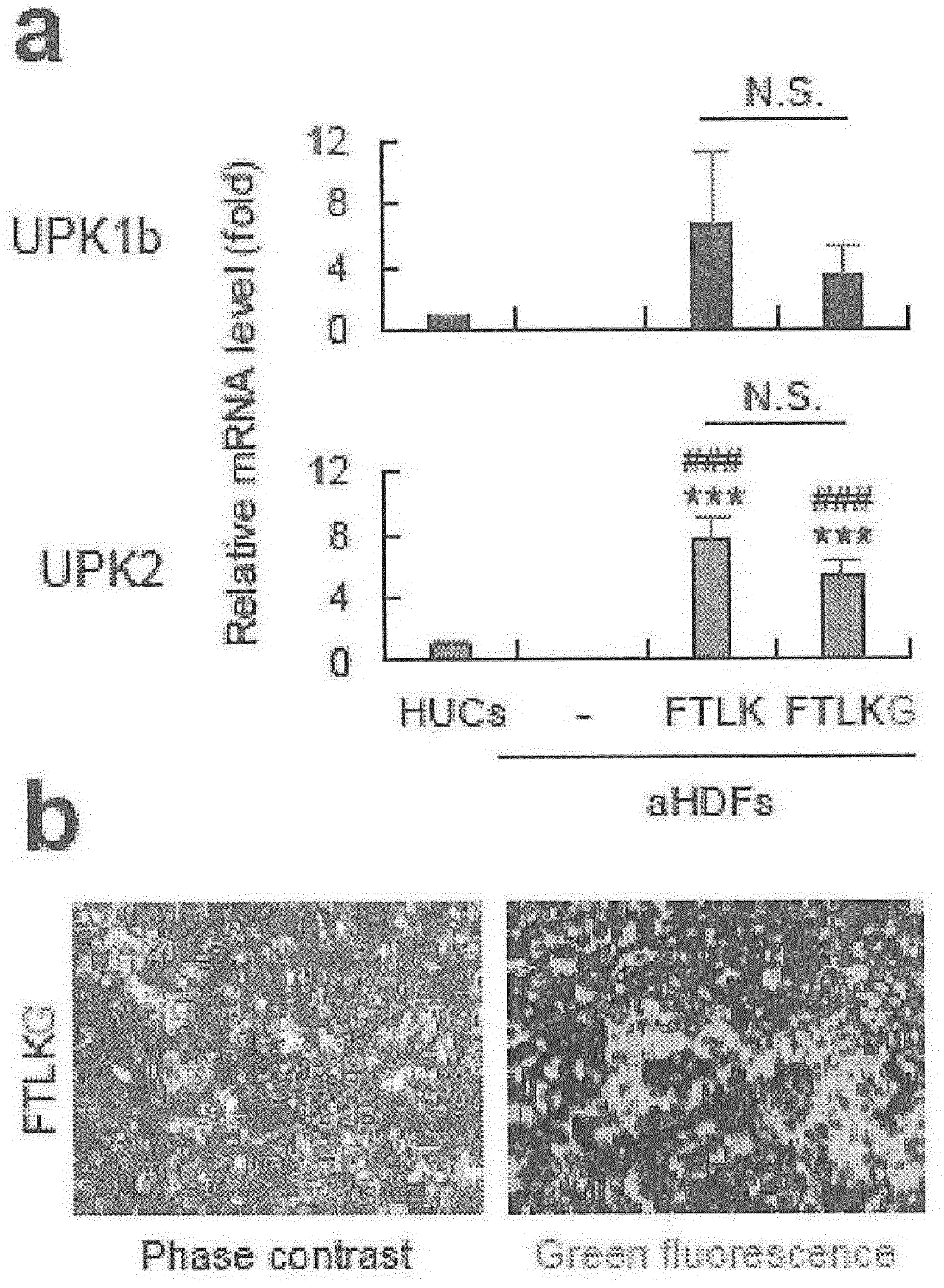
a
N.S.
UPK1b
Relative mRNA level (fold)
12
8
4
0
N.S.
UPK2
###
***
###
***
12
8
4
0
HUCs
-
FTLK
FTLKG
aHDFs
b
FTLKG
Phase contrast
Green fluorescence

FIG. 18B

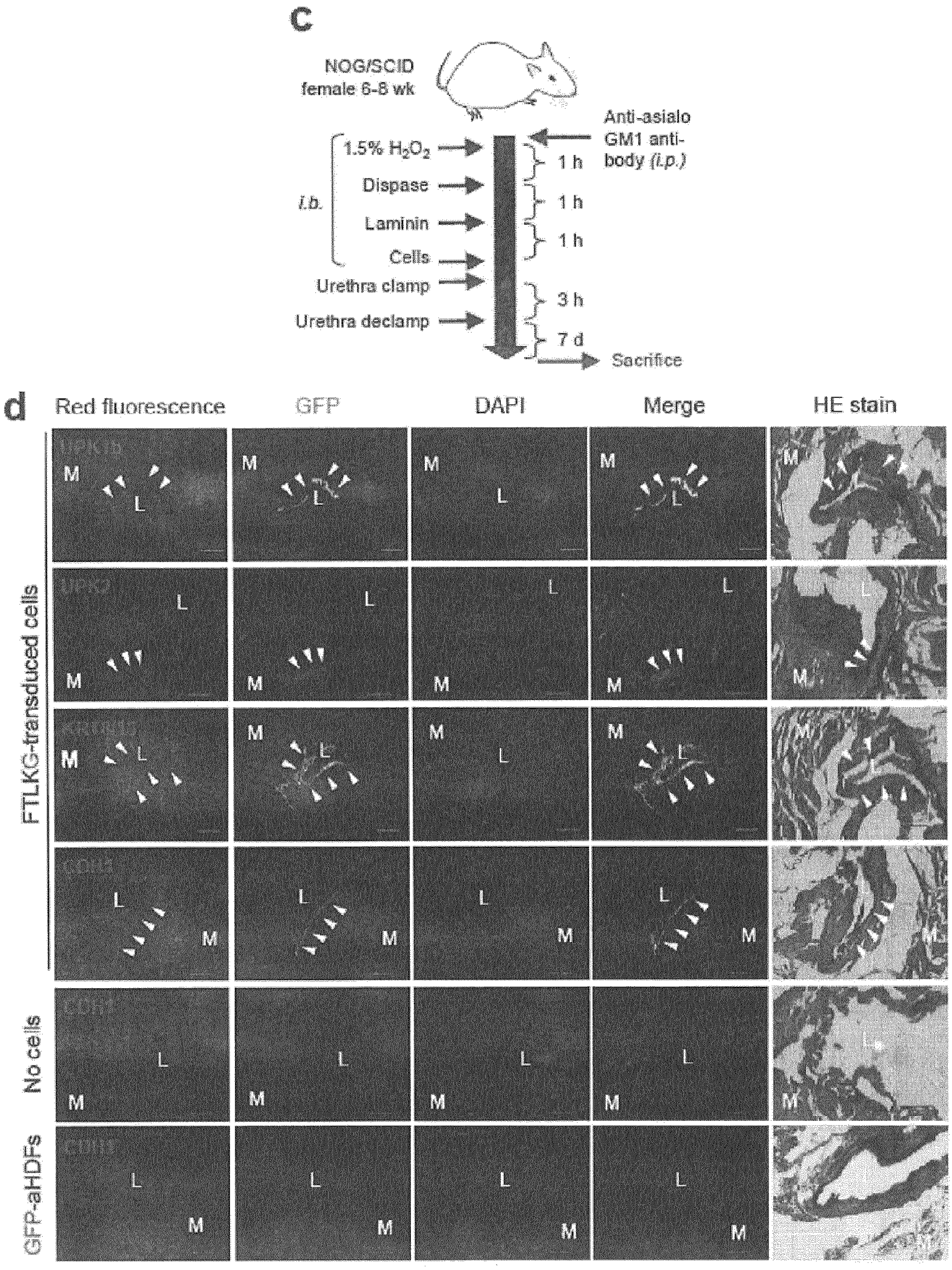
c
NOG/SCID
female 6-8 wk
Anti-asialo
GM1 anti-
body (i.p.)
i.b.
1.5% $H_2O_2$
Dispase
Laminin
Cells
Urethra clamp
Urethra declamp
1 h
1 h
1 h
3 h
7 d
Sacrifice
d
Red fluorescence
GFP
DAPI
Merge
HE stain
FTLKG-transduced cells
No cells
GFP-aHDFs

FIG. 19

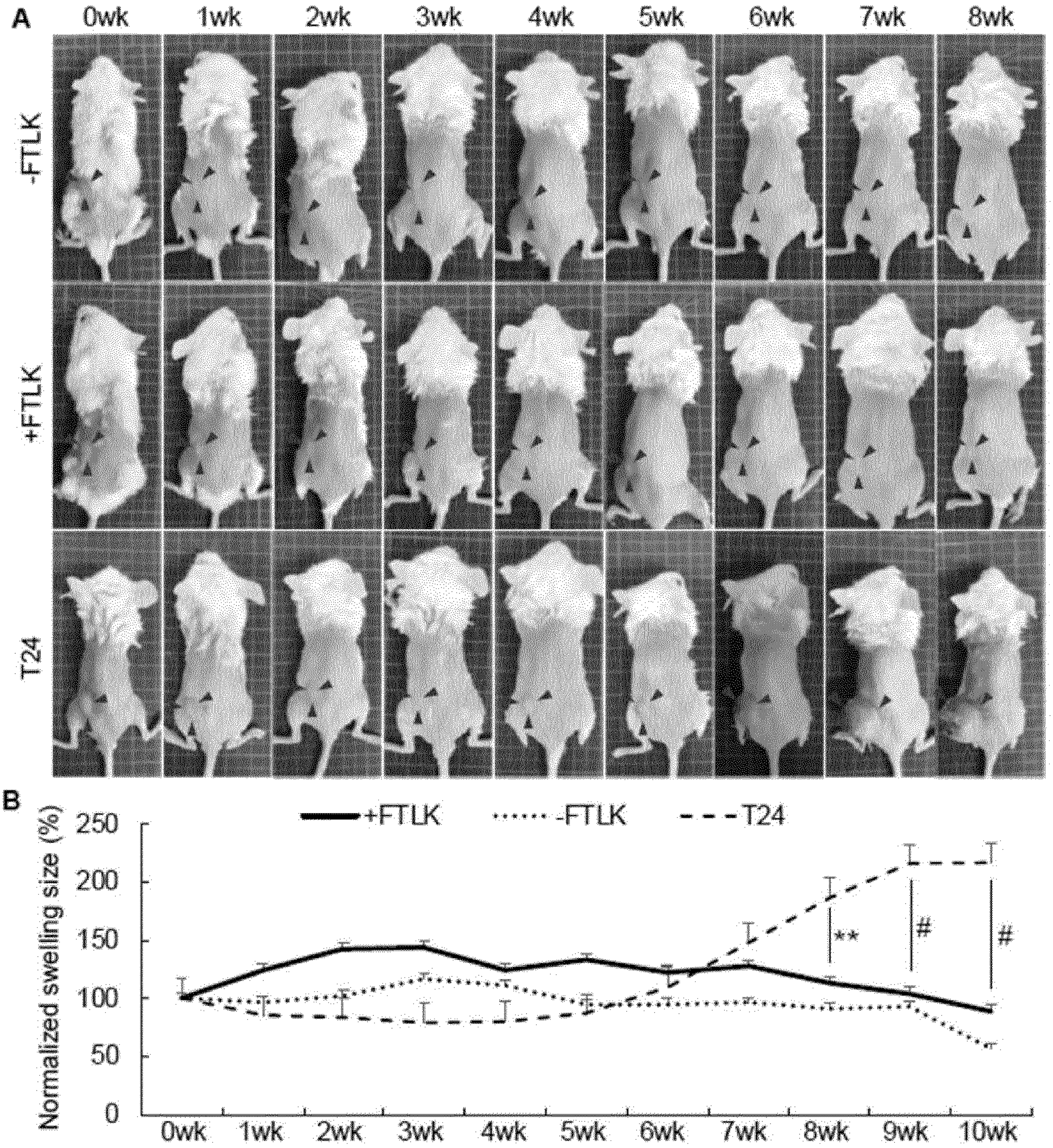
A
0wk
1wk
2wk
3wk
4wk
5wk
6wk
7wk
8wk
-FTLK
+FTLK
T24
B
+FTLK
-FTLK
T24
Normalized swelling size (%)
250
200
150
100
50
0
0wk
1wk
2wk
3wk
4wk
5wk
6wk
7wk
8wk
9wk
10wk
**
#
#

FIG. 20

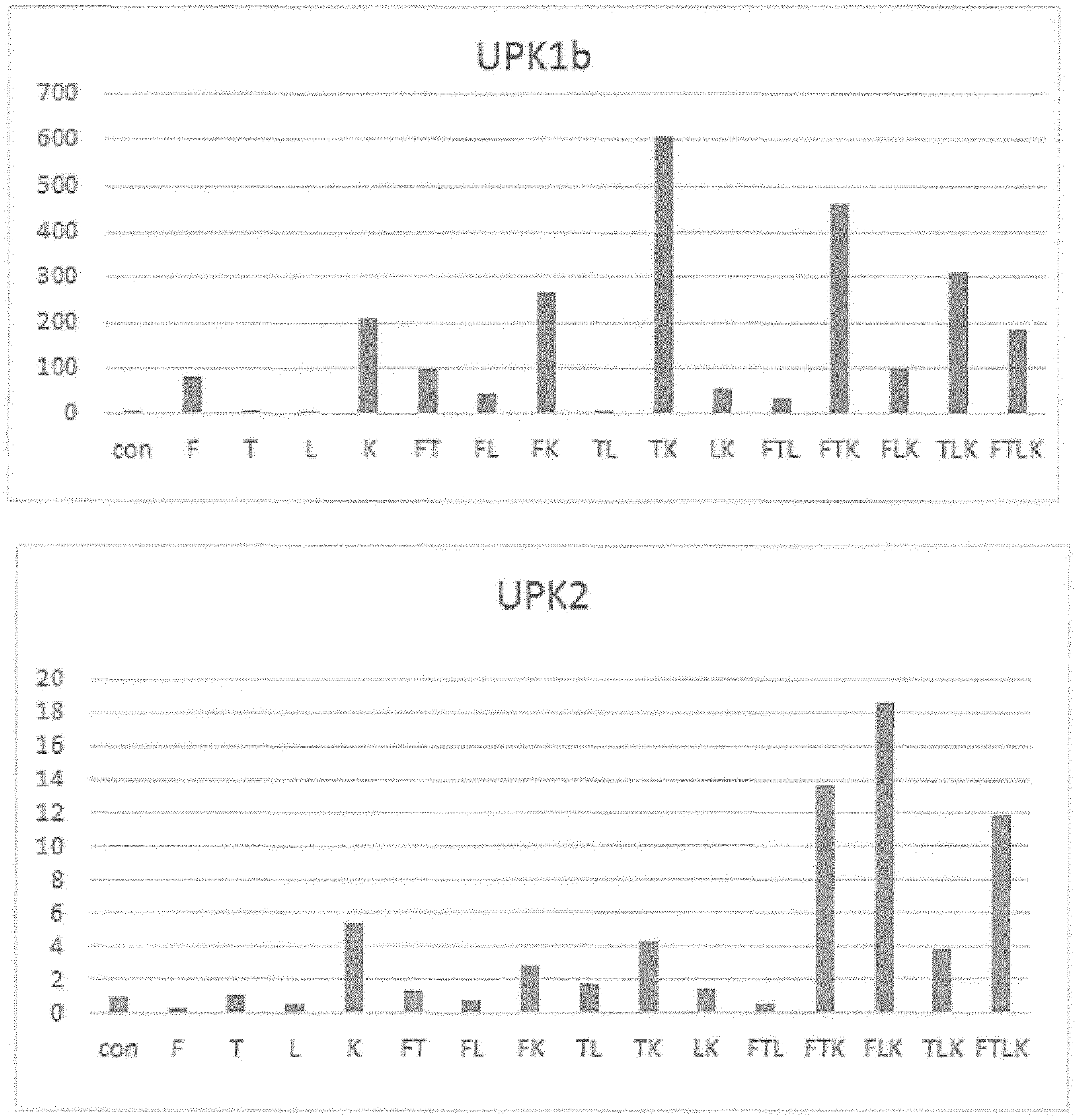
UPK1b
700
600
500
400
300
200
100
0
con F T L K FT FL FK TL TK LK FTL FTK FLK TLK FTLK
UPK2
20
18
16
14
12
10
8
6
4
2
0
con F T L K FT FL FK TL TK LK FTL FTK FLK TLK FTLK

FIG. 21

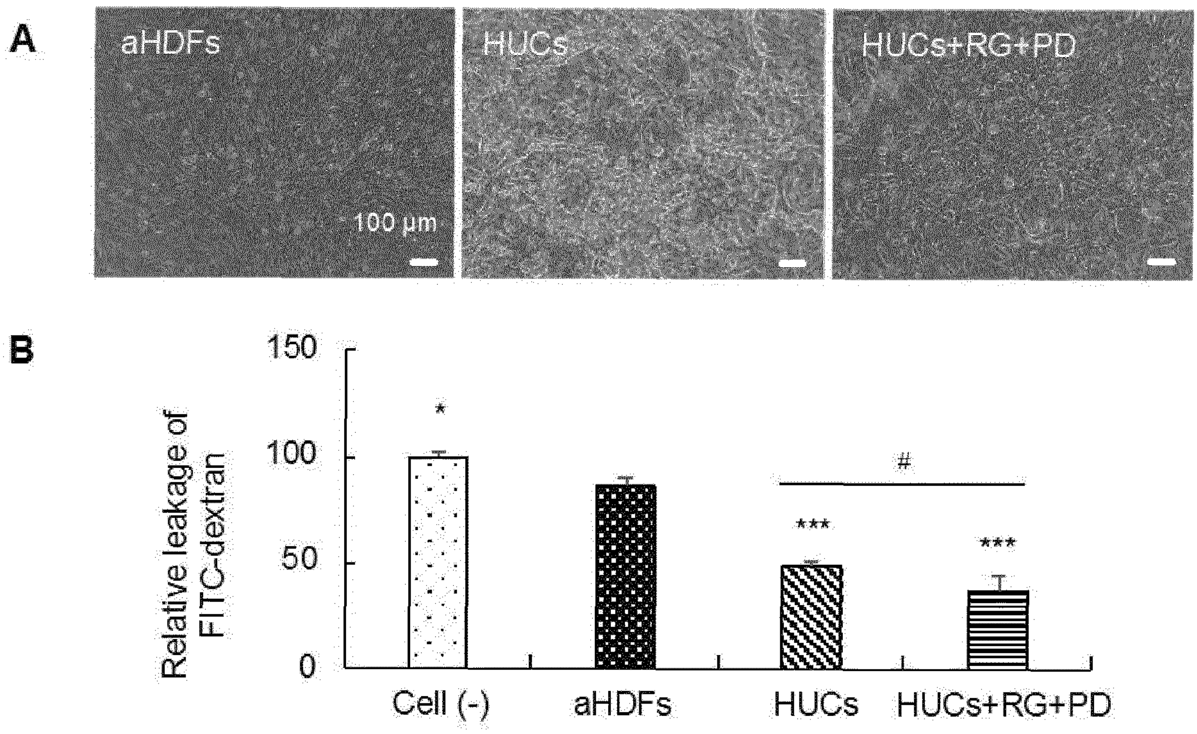
A
aHDFs
100 µm
HUCs
HUCs+RG+PD
B
150
100
50
0
Relative leakage of FITC-dextran
*
#
***
***
Cell (-)
aHDFs
HUCs
HUCs+RG+PD

## INTERNATIONAL SEARCH REPORT

International application No.
PCT/JP2020/032644

A. CLASSIFICATION OF SUBJECT MATTER
A61K 48/00(2006.01)i; A61P 13/00(2006.01)i; A61P 13/02(2006.01)i; A61P 13/10(2006.01)i; A61P 35/00(2006.01)i; A61P 43/00(2006.01)i; C12N 5/10(2006.01)i; C12N 15/12(2006.01)i; C12N 15/85(2006.01)i; A61K 35/22(2015.01)i; A61K 35/76(2015.01)i
FI: C12N15/85 Z; C12N15/12; A61K35/22; A61K35/76; A61K48/00; A61P43/00 105; A61P13/00; A61P13/02; A61P13/10; A61P35/00; C12N5/10 ZNA
According to International Patent Classification (IPC) or to both national classification and IPC

B. FIELDS SEARCHED
Minimum documentation searched (classification system followed by classification symbols)
A61K48/00; A61P13/00; A61P13/02; A61P13/10; A61P35/00; A61P43/00; C12N5/10; C12N15/12; C12N15/85; A61K35/22; A61K35/76

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLlNE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>A<br>Y | WO 2013/130769 A1 (THE TRUSTEES OF COLUMBIA UNIVERSITY IN THE CITY OF NEW YORK) 06 September 2013 (2013-09-06) claims, examples 5-6 | 5, 7-8, 13, 15-19<br>1-4, 6, 9-12,14<br>5, 7-8, 13, 15-19 |
| Y | US 2018/0346933 A1 (THE RECENTS OF THE UNIVERSITY OF COLORADO, A BODY CORPORATE) 06 December 2018 (2018-12-06) paragraph [0083] | 5, 7-8, 13, 15-19 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 October 2020 (16.10.2020) | 10 November 2020 (10.11.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/032644

C (Continuation). DOCUMENTS CONSIDERED TO BE RELEVANT

| Category* | Citation of document, with indication, where appropriate, of the relevant passages | Relevant to claim No. |
|---|---|---|
| X<br>A | KANG, Min-Yong et al., "Generation of Bladder Urothelium from Human Pluripotent Stem Cells under Chemically Defined Serum- and Feeder-Free System", International Journal of Molecular Sciences, 2014, vol. 15, pp. 7139-7157, DOI: 10.3390/ijms15057139 fig. 1B, 3A | 5, 8, 17-19<br>6-7 |
| X<br>A | HUSTLER, Arianna et al., "Differential transcription factor expression by human epithelial cells of buccal and urothelial derivation", Experimental Cell Research, 2018, vol. 369, pp. 284-294, DOI:10.1016/j.yexcr.2018.05.031 ABSTRACT, fig. 3C, 4A-C | 5, 8, 17-19<br>6-7 |
| X<br>A | VARLEY, CL et al., "FOXA1 and IRF-1 intermediary transcriptional regulators of PPARγ-induced urothelial cytodifferentiation", Cell Death and Differentiation, 2009, vol. 16, pp. 103-114, DOI:10.1038/cclcl.2008.116 ABSTRACT, fig. 1-3 | 5, 8, 17-19<br>6-7 |
| A | WO 2017/069222 A1 (KYOTO PREFECTURAL PUBLIC UNIVERSITY CORPORATION) 27 April 2017 (2017-04-27) claims, paragraphs [0060], [0063], [0067]-[0068], [0086] | 20 |
| A | OSBORN, Stephanie L. et al., "Induction of Human Embryonic and induced Pluripotent Stem Cells Into Urothelium", STEM CELLS TRANSLATIONAL MEDICINE, 2014, vol. 3, pp. 610-619, DOI: 10.5966/sctm.2013-0131 ABSTRACT, MATERIALS AND METHODS | 20 |
| P, X | INOUE, Yuta et al., "Direct conversion of fibroblasts into urothelial cells that may be recruited to regenerating mucosa of injured urinary bladder", SCIENTIFIC REPORTS, 25 September 2019, vol. 9, 13850, DOI: 10.1038/s41598-019-50388-6 entire text | 1-20 |

Form PCT/ISA/210 (continuation of second sheet) (January 2015)

**INTERNATIONAL SEARCH REPORT**
Information on patent family members

International application No.
PCT/JP2020/032644

| Patent Documents referred in the Report | Publication Date | Patent Family | Publication Date |
|---|---|---|---|
| WO 2013/130769 A1 | 06 Sep. 2013 | US 2015/0023934 A1<br>claims, examples 5-6 | |
| US 2018/0346933 A1 | 06 Dec. 2018 | WO 2017/091547 A1<br>paragraph [0085]<br>EP 3384038 A1<br>CA 3011692 A | |
| WO 2017/069222 A1 | 27 Apr. 2017 | US 2019/0024054 A1<br>claims, paragraphs [0107], [0110], [0114]-[0115], [0145]<br>EP 3378933 A1<br>KR 10-2018-0073631 A<br>CN 109072183 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015012377 A
- WO 2014010746 A
- WO 2016039462 A
- WO 2018124292 A
- JP 2019159070 A


### Non-patent literature cited in the description


- **NING J et al.** *Cytotechnology,* 2011, vol. 63 (5), 531-539
- **SHI JG et al.** *Cell Tissue Res,* 2012, vol. 347 (3), 737-746
- **OSBORN SL et al.** *Stem Cell Transl Med,* 2014, vol. 3 (5), 610-619
- **KANG M et al.** *Int J Mol Sci,* 2014, vol. 15 (5), 7139-7157
- **YAMAMOTO K et al.** *Proc Natl Acad Sci USA,* 2015, vol. 112 (19), 6152-6157
- **KISHIDA T et al.** *Stem Cell Reports,* 2015, vol. 5 (4), 569-581
- **SOWA Y et al.** *Stem Cells Transl Med,* 2017, vol. 6 (4), 1207-1216
- **WAKAO J et al.** *Biochem Biophys Res Commun,* 2017, vol. 488 (2), 368-373
- **MASAKI IEDA ; JI-DONG FU ; PAUL DELGADO-OLGUIN ; VASANTH VEDANTHAM ; YOHEI HAYASHI ; BENOIT G. BRUNEAU ; DEEPAK SRIVASTAVA.** Direct Reprogramming of Fibroblasts into Functional Cardiomyocytes by Defined Factors. *Cell,* 2010, vol. 142, 375-386
- **THOMAS VIERBUCHEN ; AUSTIN OSTERMEIER ; ZHIPING P. PANG ; YUKO KOKUBU ; THOMAS C. SUDHOF ; MARIUS WERNIG.** Direct conversion of fibroblasts to functional neurons by defined factors. *Nature,* vol. 46
- **PANG ZP ; YANG N ; VIERBUCHEN T ; OSTERMEIER A ; FUENTES DR ; YANG TQ ; CITRI A ; SEBASTIANO V ; MARRO S ; SUDHOF TC.** Induction of human neuronal cells by defined transcription factors. *Nature,* 2011, vol. 476, 220-223
- **KUNIHIKO HIRAMATSU ; SATORU SASAGAWA ; HIDETATSU OUTANI ; KANAKO NAKAGAWA ; HIDEKI YOSHIKAWA ; NORIYUKI TSUMAKI.** Generation of hyaline cartilaginous tissue from mouse adult dermal fibroblast culture by defined factors. *Journal of Clinical Investigation,* 2011, vol. 121, 640-657
- **PENGYU HUANG ; ZHIYING HE ; SHUYI JI ; HUAWANG SUN ; DAO XIANG ; CHANGCHENG LIU ; YIPING HU ; XINWANG ; LIJIAN HUI.** Induction of functional hepatocyte-like cells from mouse fibroblasts by defined factors. *Nature,* 2011, vol. 475, 386-389
- **SAYAKA SEKIYA ; ATSUSHI SUZUKI.** Direct conversion of mouse fibroblasts to hepatocyte-like cells by defined factors. *Nature,* 2011, vol. 475, 390-393
- **YAMAMOTO K ; KISHIDA T ; SATO Y ; NISHIOKA K ; EJIMA A ; FUJIWARA H ; KUBO T ; YAMAMOTO T ; KANAMURA N ; MAZDA O.** Direct conversion of human fibroblasts into functional osteoblasts by defined factors. *Proc Natl Acad Sci USA.,* 2015, vol. 112, 6152-6157
- **KISHIDA T ; EJIMA A ; YAMAMOTO K ; TANAKA S ; YAMAMOTO T ; MAZDA O.** Reprogrammed Functional Brown Adipocytes Ameliorate Insulin Resistance and Dyslipidemia in Diet-Induced Obesity and Type 2 Diabetes. *Stem Cell Reports,* 2015, vol. 5, 569-581
- **YAMAMOTO K. ; SATO Y. ; HONJO K. ; ICHIOKA H. ; OSEKO F. ; SOWA Y. ; YAMAMOTO T. ; KANAMURA N. ; KISHIDA T. ; MAZDA O.** Generation of directly converted human osteoblasts that are free of exogenous gene and xenogenic protein. *J Cell Biochem,* 2016, vol. 117, 2538-2545
- **OSBORN S.L. et al.** *Stem Cells Transl Med.,* 2014, vol. 3 (5), 610-619
- **KANG M. et al.** *Int. J. Mol. Sci.,* 2014, vol. 15 (5), 7139-7157
- **IEDA M.** Heart regeneration using reprogramming technology. *Proc Jpn Acad Ser B Phys Biol Sci.,* 2013, vol. 89 (3), 118-28
- **IEDA M ; FU JD ; DELGADO-OLGUIN P ; VEDANTHAM V ; HAYASHI Y ; BRUNEAU BG ; SRIVASTAVA D.** Direct reprogramming of fibroblasts into functional cardiomyocytes by defined factors. *Cell,* 06 August 2010, vol. 142 (3), 375-86

- **QIAN L ; HUANG Y ; SPENCER CI ; FOLEY A ; VEDANTHAM V ; LIU L ; CONWAY SJ ; FU JD ; SRIVASTAVA D.** In vivo reprogramming of murine cardiac fibroblasts into induced cardiomyocytes. *Nature,* 31 May 2012, vol. 485 (7400), 593-8
- **OSBORN SL.** *Stem Cell Transl Med,* 2014, vol. 3 (5), 610-619
- **NAKAGAWA M et al.** *Sci Rep,* 2014, vol. 4 (3594
- **LIAO, Y. ; SMYTH, G. K. ; SHI, W.** featureCounts: an efficient general purpose program for assigning sequence reads to genomic features. *Bioinformatics,* 2014, vol. 30, 923-930
- **GE, S. X. ; SON, E. W. ; YAO, R.** iDEP: an integrated web application for differential expression and pathway analysis of RNA-Seq data. *BMC Bioinformatics,* 2018, vol. 19
- **KANEHISA, M. ; FURUMICHI, M. ; TANABE, M. ; SATO, Y. ; MORISHIMA, K.** KEGG: new perspectives on genomes, pathways, diseases and drugs. *Nucleic Acids Res,* 2017, vol. 45, D353-D361
- **LUO, W. ; BROUWER, C.** Pathview: an R/Bioconductor package for pathway-based data integration and visualization. *Bioinformatics,* 2013, vol. 29, 1830-1831
- **HOMAN T et al.** *J Pharmacol Sci,* 2013, vol. 121 (4), 327-337
- **SHIMIZU T et al.** *Oncoimmunology,* 2018, vol. 7 (5), e1424671
- **KANDA, Y.** Investigation of the freely available easy-touse software 'EZR' for medical statistics. *Bone. Marrow. Transplant.,* 2013, vol. 48, 452-458
- **HABUKA, M. et al.** *PLoS. One.,* 2015, vol. 10, e0145301